# EUROPEAN PATENT APPLICATION

(11) **EP 4 559 992 A1**
(43) Date of publication of application: **28.05.2025**
(21) Application number: 23857414.9
(22) Date of filing: 24.08.2023
(51) Int. Cl.: C09K 11/06, A61M 5/28, A61K 49/00, A61M 37/00

(54) **COMPOSITION FOR FLUORESCENT LABELING, FLUORESCENT PROBE, INJECTION AGENT, SYRINGE FILLING, MEDICAL APPARATUS, MEDICAL FIBER MATERIAL, METHOD FOR PRODUCING COMPOSITION FOR FLUORESCENT LABELING, AND METHOD FOR PRODUCING MEDICAL FIBER MATERIAL**

(30) Priority: 24.08.2022 JP 2022133013
(71) Applicant: Vita Corporation, Kobe-shi, Hyogo 651-0084 (JP); National University Corporation Kobe University, Kobe-shi, Hyogo 657-8501 (JP)
(72) Inventor: TSUCHIDA Shinobu, Kobe-shi, Hyogo 650-0004 (JP); URADE Takeshi, Kobe-shi, Hyogo 657-8501 (JP)
(74) Representative: Isarpatent
(86) International application number: PCT/JP2023/030491
(87) International publication number: WO 2024/043306

(57) **Abstract**

A composition for fluorescent labeling contains a compound (A) that is an ICG compound and a hydroxyl group-containing organic compound (B). In this way, it is possible to provide a composition for fluorescent labeling that has excellent compartment identifiability to identify an observation target more accurately, a long light emission time to observe the observation target for a long time, a small load to a biological body, and good economic efficiency.

## Description

### Technical Field

The present invention relates to a composition for fluorescent labeling, a fluorescent probe, an injection agent, a syringe filling, a medical apparatus, a medical fiber material, a method for producing composition for fluorescent labeling, and a method for producing medical fiber material.

### Background Art

As a method of visualizing and observing a surface or interior state of a biological body tissue of a blood vessel, a lymph vessel, an organ, or the like, biological fluorescence imaging is used. The biological fluorescence imaging is a technique for visualizing fluorescent light emission of a specific protein or cell organelle, which is labeled (marked) with a fluorescent labeling compound, using a fluorescent microscope, camera, or the like. As the fluorescent labeling compound used for the biological fluorescence imaging, indocyanine green (ICG) is used, for example.

The biological fluorescence imaging using ICG is clinically used in surgery field, for example. For instance, ICG is injected into a biological body, and is irradiated with excitation light so as to generate fluorescent light, so that an observation target of a fluorescent contrast-enhanced part or the like is imaged and monitored. In this way, the state of the blood vessel, lymph vessel, organ, or the like can be non-invasively observed.

An example of a technique related to this biological fluorescence imaging is disclosed in Patent Document 1. Patent Document 1 discloses a multipurpose medical imaging marker containing a rubber material and a fluorescent dye. As the fluorescent dye, ICG is used.

On the other hand, Patent Document 2, for example, discloses a method for producing ICG-containing particles aimed at containing ICG as monomers in large quantity in particles. The above producing method includes the step of mixing ICG, particles, and a solution containing a chaotropic agent with a concentration of 1 mM to 10 M.

Further, examples of fluorescent labeling compounds other than ICG are disclosed in Patent Documents 3 and 4. More specifically, Patent Documents 3 and 4 disclose thermoplastic resin compositions containing a near-infrared fluorescent dye consisting of an azo-boron complex compound, as a resin composition that emits near-infrared fluorescent when irradiated by excitation light in a near-infrared region, and can be detected by a detector.

### List of Citations

### Patent Literature

Patent Document 1: JP-A-2019-167341
Patent Document 2: JP-A-2014-227338
Patent Document 3: JP-A-2015-025105
Patent Document 4: WO2013/180127

### Summary of the Invention

### Technical Problem

In the technique disclosed in Patent Document 1, dimethyl sulfoxide (DMSO) or the like is used as a solvent of ICG. As DMSO is a solvent that is highly irritable to skin or mucous membrane, and is also highly permeable to skin, it is preferred not to use it as much as possible. Further, DMSO has cytotoxicity, which is also the reason why use of DMSO should be avoided as much as possible.

The technique disclosed in Patent Document 2 is directed to containing ICG as monomers without coagulation, in a liposome at high concentration. However, in Patent Document 2, there is no discussion about compartment identifiability of fluorescent light emission for accurately identifying an observation target such as a fluorescent contrast-enhanced part, or light emission time for observing the observation target for a long time.

The technique disclosed in Patent Document 3 or 4 uses an azo-boron complex compound, and is not preferred because of toxicity. In addition, it is a new compound that is not generally used in clinic or the like, and hence it needs cost for quantity synthesis and is not good in economic efficiency.

In other words, in the above biological fluorescence imaging using ICG, it is requested to have good compartment identifiability to the extent that enables to accurately identify a structure, a shape, and a state of the observation target such as the fluorescent contrast-enhanced part, and to have a long light emission time for observing the observation target for a long time. However, when ICG is administered into the biological body, it is difficult to control the light emission range, the light emission timing, and the like, resulting in insufficient fluorescent in some cases, and the above request is not sufficiently supported. For instance, if the fluorescent is insufficient when ICG is injected intravenously or administered in clinic, the operative field state may not be accurately recognized, or it may be necessary to additionally inject ICG intravenously. In addition, if the fluorescent is excessive, the operator may have to wait until ICG is metabolized and eliminated.

Furthermore, the biological fluorescence imaging is also required to be little toxic, to have a small load to a biological body, and to have good economic efficiency with reduced cost for producing the fluorescent labeling compound.

However, a technique that fully satisfies these requests has not been developed yet.

The present invention has been made in view of this situation, and its object is to provide a composition for fluorescent labeling, a fluorescent probe, an injection agent, a syringe filling, a medical apparatus, a medical fiber material, a method for producing composition for fluorescent labeling, and a method for producing medical fiber material, which have good compartment identifiability to the extent that enables to accurately identify an observation target, a long light emission time for observing the observation target for a long time, a small load to a biological body, and good economic efficiency.

### Means for Solving the Problem

A composition for fluorescent labeling according to an aspect of the present invention contains:
a compound (A) expressed by the following formula (1); and
a hydroxyl group-containing organic compound (B). 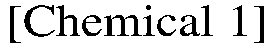 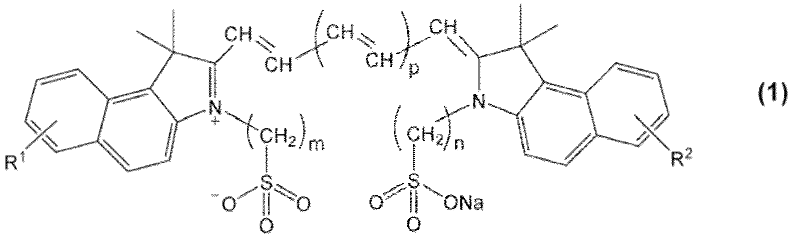 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

A fluorescent probe according to another aspect of the present invention includes the above composition for fluorescent labeling.

An injection agent according to still another aspect of the present invention is an injection agent for labeling, which labels a target existing on mucous membrane or skin, and contains the above composition for fluorescent labeling.

A syringe filling according to still another aspect of the present invention includes the above composition for fluorescent labeling and a syringe in which the composition for fluorescent labeling is filled.

A medical apparatus according to still another aspect of the present invention contains the above composition for fluorescent labeling.

A medical fiber material according to still another aspect of the present invention contains:
a compound (A) expressed by the following formula (1); and
a fiber material containing a hydroxyl group-containing organic compound (B). 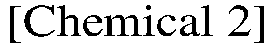 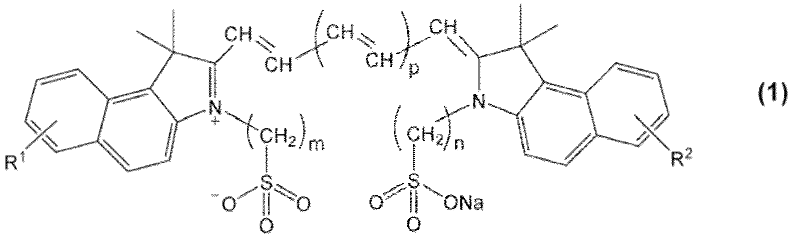 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

A method for producing composition for fluorescent labeling according to still another aspect of the present invention includes a mixing step for mixing a compound (A) expressed by the following formula (1) and a hydroxyl group-containing organic compound (B). 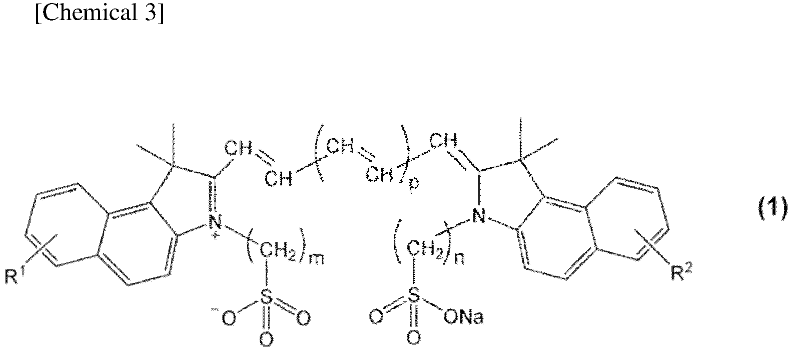 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

A method for producing medical fiber material according to still another aspect of the present invention includes:
an impregnation step of allowing a fiber material containing a hydroxyl group-containing organic compound (B) to be impregnated or sprayed with a compound (A) expressed by the following formula (1); and
a mordanting step of using a mordant for fixing the compound (A) to the fiber material. 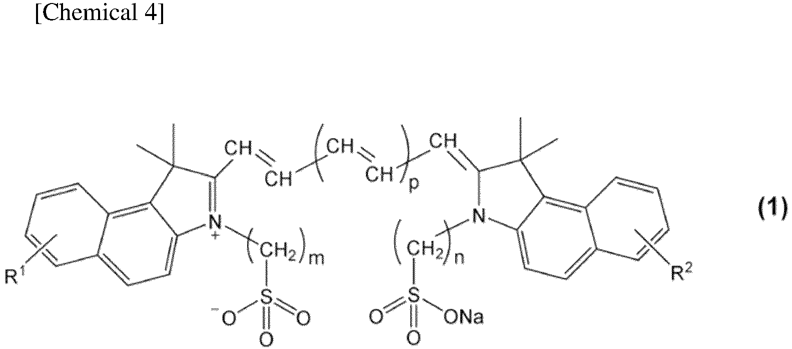 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

### Advantageous Effects of the Invention

According to the present invention, it is possible to provide a composition for fluorescent labeling, a fluorescent probe, an injection agent, a syringe filling, a medical apparatus, a medical fiber material, a method for producing composition for fluorescent labeling, and a method for producing medical fiber material, which have good compartment identifiability to the extent that enables to accurately identify an observation target, a long light emission time for observing the observation target for a long time, a small load to a biological body, and good economic efficiency.

### Brief Description of Drawings

FIG. 1A is an explanatory diagram schematically illustrating a state of arranged containers containing undiluted solutions.
FIG. 1B is an explanatory diagram illustrating near-infrared images obtained by irradiating the containers of FIG. 1A with near-infrared light.
FIG. 2 is an explanatory diagram illustrating the near-infrared image obtained for G solution.
FIG. 3 is an explanatory diagram illustrating the near-infrared image obtained for E solution.
FIG. 4 is an explanatory diagram illustrating the near-infrared image obtained for N solution.
FIG. 5 is an explanatory diagram illustrating the near-infrared image obtained for A solution.
FIG. 6A is an explanatory diagram schematically illustrating a state of arranged gauzes impregnated with diluents.
FIG. 6B is an explanatory diagram illustrating near-infrared images obtained by irradiating the gauzes of FIG. 6A with near-infrared light.
FIG. 7A is an explanatory diagram schematically illustrating an image obtained by placing a gauzes impregnated with G solution diluent in an abdominal cavity of a pig, and by imaging with an endoscopic camera.
FIG. 7B is an explanatory diagram illustrating a near-infrared image of the gauze of FIG. 7A.
FIG. 8A is an explanatory diagram schematically illustrating an image obtained by placing a gauze impregnated with E solution diluent in the above abdominal cavity, and by imaging with the endoscopic camera.
FIG. 8B is an explanatory diagram illustrating a near-infrared image of the gauze of FIG. 8A.
FIG. 9A is an explanatory diagram schematically illustrating an image obtained by placing a gauze impregnated with N solution diluent in the above abdominal cavity, and by imaging with the endoscopic camera.
FIG. 9B is an explanatory diagram illustrating a near-infrared image of the gauze of FIG. 9A.
FIG. 10A is an explanatory diagram schematically illustrating an image obtained by placing a gauze impregnated with A solution diluent in the above abdominal cavity, and by imaging with the endoscopic camera.
FIG. 10B is an explanatory diagram illustrating a near-infrared image of the gauze of FIG. 10A.
FIG. 11 is a perspective view illustrating a structural example of a CV port.
FIG. 12 is a cross-sectional view of the above CV port.
FIG. 13 is an exploded perspective view of the above CV port.
FIG. 14A is an explanatory diagram schematically illustrating a state of arranged rings made by molding epoxy resin.
FIG. 14B is an explanatory diagram illustrating near-infrared images of the rings of FIG. 14A.
FIG. 15 is an explanatory diagram illustrating near-infrared images of the rings made by molding with adding solution containing ICG at a concentration four times higher than that of the rings of FIG. 14A.
FIG. 16A is an explanatory diagram schematically illustrating a state of arranging the above CV port and a comparison port.
FIG. 16B is an explanatory diagram illustrating a near-infrared image of the above CV port including an index member molded using G solution.
FIG. 17 is an explanatory diagram illustrating a near-infrared image of the above CV port including an index member molded using E solution.
FIG. 18 is an explanatory diagram illustrating a near-infrared image of the above CV port including an index member molded using N solution.
FIG. 19 is an explanatory diagram illustrating a near-infrared image of the above CV port including an index member molded using A solution.

### Description of Embodiments

Hereinafter, an embodiment for implementing the present invention (hereinafter, simply referred to as "the present embodiment") is described in detail. The following present embodiment is an example for describing the present invention, and it is not intended to limit the present invention to the following description. The present invention can be appropriately modified for implementation within the scope of the spirit thereof. Note that in the following description, when expressing a value range in a form of AA to BB, the values of the lower limit AA and the upper limit BB are included in the value range.

### <Composition for Fluorescent Labeling>

The composition for fluorescent labeling according to the present embodiment is a composition for fluorescent labeling that contains a compound (A) expressed by the following formula (1), and a hydroxyl group-containing organic compound (B). The compound (A) contains indocyanine green ("ICG") and its derivative ("indocyanine green derivative", "ICG derivative"). Note that in this specification, ICG and ICG derivative may be generically referred to as "indocyanine green compound" ("ICG compound") or the like. 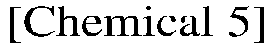 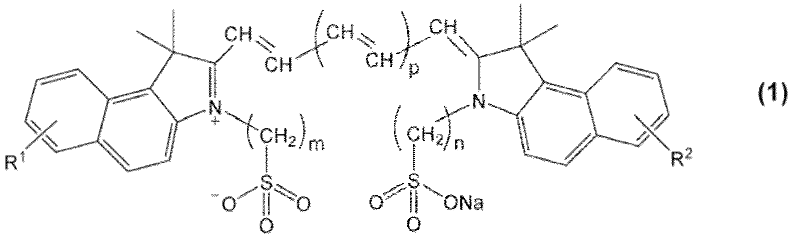 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

### ((A) Component: Indocyanine Green Compound)

The compound (A) expressed by the formula (1) contains indocyanine green (ICG). ICG is a compound (CAS No. 3599-32-4) having a structure expressed by the following formula (1a), and is a fluorescent organic dye whose excitation light and fluorescent light are both in the near-infrared region. The formula (1a) is the case where R¹ and R² are both hydrogen, p is 2, and m and n are both 4 in the formula (1).

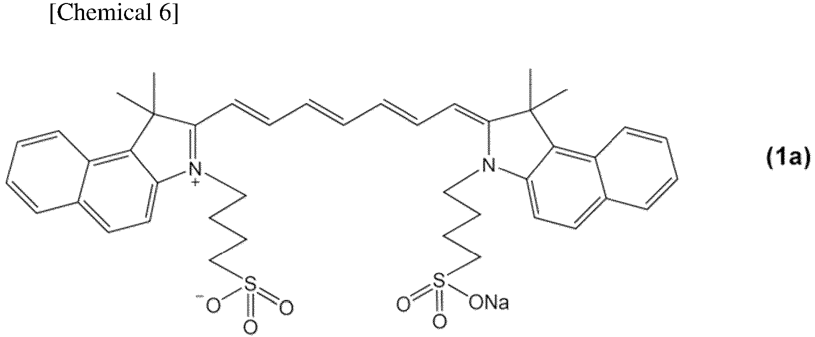

R¹ and R² independently represent hydrogen, an alkyl group of carbon number 1 to 13, an alkoxy group of carbon number 1 to 13, or a sulfonate group, and they independently and preferably represent hydrogen, an alkyl group of carbon number 1 to 8, an alkoxy group of carbon number 1 to 8, more preferably hydrogen, an alkyl group of carbon number 1 to 4, an alkoxy group of carbon number 1 to 4, and still more preferably hydrogen.

Symbol p represents an integer of 1 to 5, preferably 1 to 4, more preferably 2 to 3, and still more preferably 2.

Symbols m and n independently represent an integer of 1 to 12, preferably 1 to 8, more preferably 2 to 6, still more preferably 3 to 5, and still more preferably 4.

ICG absorbs little excitation energy of wavelengths of 600 nm or less, and absorbs excitation energy of wavelengths of 650 to 900 nm so as to generate fluorescent light. For this reason, it is difficult to visually identify fluorescent light when irradiated with visible light, but fluorescent light generated when irradiated with near-infrared light as excitation light can be easily visualized by receiving it with a fluorescent microscope, a camera, or the like. Therefore, when the composition for fluorescent labeling according to the present embodiment is used for biological fluorescence imaging in the blood, ICG excitation light is not absorbed by hemoglobin or the like having high absorption in the visible light region of 600 nm or less. Therefore, it is possible to allow the ICG excitation light to reach deep inside of the biological body tissue, and it has high biological penetrability. Thus, it can be said that ICG is a near-infrared fluorescent organic dye that is suitable for observing a biological body tissue, i.e., for observing a state or the like of a blood vessel, a lymph vessel, or an organ.

However, ICG may be unstable in fluorescent coloring, or may not be able to obtain sufficient fluorescence intensity, or may be deactivated in a short time. For this reason, in a case where compartment identifiability is required in clinic, such as decision of an excision area in surgery or the like, or in a case where aging and successive monitoring is needed for a long time, a conventional biological fluorescence imaging using ICG is not sufficient and has points to be improved.

In this point, according to the present embodiment, by combining ICG and a hydroxyl group-containing organic compound described later, stable fluorescent coloring can be obtained, and sufficient fluorescence intensity can be obtained, and hence it is possible to obtain good compartment identifiability to the extent that enables to more clearly identify a structure or the like of an observation target such as a fluorescent contrast-enhanced part. Further, because of a long light emission time, the observation target can be observed for a long time.

The reason why such advantages are obtained is not clear but can be conjectured as follows. By combining ICG and the hydroxyl group-containing organic compound, they can be strongly bound by hydrogen bond action and can obtain a stable form. As a result, it is considered that good compartment identifiability and long light emission time can be achieved. (However, actions of the present embodiment are not limited to these.) As the above hydrogen bond, for example, a bond between a hydrogen atom contained in the hydroxyl group of the hydroxyl group-containing organic compound and an oxygen atom contained in ICG can be considered.

Note that the situation described above can also be applied to a case using an ICG derivative having the same basic skeleton as ICG (see the formula (1a)), excepting the case where R¹ and R² are both hydrogen, p is 1, and m and n are both 4 in the formula (1).

Note that, as the fluorescent organic dye, it is preferred to contain only the compound (the ICG compound) expressed by the formula (1). In other words, the fluorescent organic dye preferably contains any one of ICG, an ICG derivative, and both of them. For instance, in view of toxicity, economic efficiency, or the like, it is preferred that an azo-boron complex compound disclosed in Patent Document 3 or 4 is not contained substantially. Note that in this specification, "not contained substantially" or "not mixed substantially" means that the component is not added or mixed positively, and it is not intended to exclude that the component is inevitably added or mixed. The most desirable case of "not contained substantially" is a case where the content is zero.

### ((B) Component: Hydroxyl Group-containing Organic Compound)

The hydroxyl group-containing organic compound (B) is an organic compound containing a hydroxyl group (OH group) as its function. As the hydroxyl group-containing organic compound (B), for example, an alcoholic hydroxyl group-containing compound (B1) can be used. In addition, when applying the above ICG compound to a medical fiber material described later, cellulose (B2) or silk (B3) can be used as the hydroxyl group-containing organic compound (B). The silk (B3) is a protein containing a hydroxyl group-containing amino acid such as tyrosine or serine as a part of the composition. Note that water is an inorganic compound and is not included in the above hydroxyl group-containing organic compound (B). Further, when applying the ICG compound to a resin material described later, a polymer such as epoxy resin (B4) in which monomers containing a hydroxyl group are polymerized can be used.

### ((B1) Component: Alcoholic Hydroxyl Group-containing Compound)

The alcoholic hydroxyl group-containing compound (B1) is not limited particularly, but is preferably an aliphatic alcohol. As an aliphatic alcohol, it is more preferred to be an aliphatic alcohol of carbon number 1 to 10. The carbon number of the aliphatic alcohol of carbon number 1 to 10 is more preferably 1 to 5, and still more preferably 1 to 4.

As a specific example of the alcoholic hydroxyl group-containing compound (B1), although not limited particularly, there are monoalcohols such as methanol, ethanol, isopropyl alcohol, and propylene glycol; diols such as diethylene glycol; triols such as glycerin; and sugar alcohols such as mannitol, for example. Among these, ethanol, isopropyl alcohol, glycerol, and the like are preferred in view of cytotoxicity and tissue damage. In particular, glycerol and the like is more preferred in view of flammability and safety to human body.

One type of the alcoholic hydroxyl group-containing compound (B1) may be used solely, or two or more types may be combined in use.

The content of the compound (A) in the composition for fluorescent labeling is not limited particularly, but it is preferably 0.0000001 to 10 wt%, more preferably 0.0000001 to 1 wt%, still more preferably 0.000001 to 1 wt%, and still more preferably 0.000001 to 0.6 wt%. If the concentration of the fluorescent dye is a certain constant concentration or more, concentration quenching phenomenon may occur in which fluorescence intensity (quantum yield) is lowered. In the present embodiment, by setting the content of the compound (A) to be in the above range, the concentration quenching phenomenon can be effectively suppressed. As a result, the compartment identifiability when fluorescent light is generated can be improved more, and the light emission time can also be increased more. (However, actions of the present embodiment are not limited to these.)

In addition, the content of the alcoholic hydroxyl group-containing compound (B1) in the composition for fluorescent labeling is not limited particularly, but is preferably 90 to 99.9999999 wt%, more preferably 99 to 99.9999999 wt%, still more preferably 99 to 99.999999 wt%, and still more preferably 99.4 to 99.999999 wt%.

Further, as a combination of contents of the compound (A) and the alcoholic hydroxyl group-containing compound (B1), it is preferred that the content of the compound (A) is 0.0000001 to 10 wt%, while the content of the alcoholic hydroxyl group-containing compound (B1) is 1 to 90 wt%. By setting the contents of the compound (A) and the alcoholic hydroxyl group-containing compound (B1) to be in the above range, the compartment identifiability when fluorescent light is generated is improved more, and the light emission time is also increased more. Note that the above contents of the compound (A) and the alcoholic hydroxyl group-containing compound (B) are contents when the composition for fluorescent labeling is used, and it may be possible to prepare the composition for fluorescent labeling at high concentration before use, and to dilute the composition for fluorescent labeling when using it, so as to realize the above content.

In addition, a content ratio of the alcoholic hydroxyl group-containing compound (B1) to total 100 mass parts of the compound (A) and the alcoholic hydroxyl group-containing compound (B1) is not limited particularly, but is preferably 97 to 99.99998, more preferably 99.96 to 99.9999, and still more preferably 99.4 to 99.9997. By setting the content ratios of the compound (A) and the alcoholic hydroxyl group-containing compound (B1) to be in the above range, the compartment identifiability when fluorescent light is generated is improved more, and the light emission time is also increased more.

Further, according to the present embodiment, it is not always necessary to use organic solvent such as DMSO or chloroform, and hence a load to a biological body is small. In addition, a commercialized product can be used as the alcoholic hydroxyl group-containing compound, which is good for economic efficiency. From this viewpoint, it is preferred that the composition for fluorescent labeling according to the present embodiment does not contain DMSO substantially. Similarly, it is preferred that it does not contain chloroform substantially. As it does not contain these organic solvents substantially, the advantages described above can be further improved. It is most preferred that contents of DMSO and chloroform are zero.

The composition for fluorescent labeling according to the present embodiment may further contain other added ingredients or the like. As such added ingredients, a component that is added to a contrast agent is considered, for example, and specifically there are sodium chloride, sodium dihydrogen phosphate, sodium hydrogenphosphate, sodium amidotrizoate, amidotrizoate sodium meglumine, iohexol, and the like.

Here, an example where the composition for fluorescent labeling according to the present embodiment is used in clinic is described.

When using for angiography, liquid of the composition for fluorescent labeling is administered into the biological body by intra-arterial injection, intravenous injection, or the like, and contrast imaging can be performed by a fluorescence observation device (such as a camera) that supports excitation light and fluorescent of the compound (A). In other words, after intravenous injection of the compound (A), the fluorescence observation device irradiates the operative field with near-infrared light (excitation light), and not only visual observation but also fluorescent observation with near-infrared light can be used. In this way, the operative field state can be accurately grasped.

For instance, when the composition for fluorescent labeling is administered in the blood, it is possible to determine where sufficient blood reaches. If the blood normally reaches, fluorescent light is generated in a relatively short time of a few seconds to a few minutes. In other words, if sufficient fluorescent light is generated, it can be determined that sufficient blood has reached. Note that the compound (A) becomes strong when it bonds with protein in the blood, and generates fluorescent light when irradiated with excitation light.

Alternatively, when using for sentinel lymph node mapping or the like, liquid of the composition for fluorescent labeling is injected into under the skin in a vicinity of tumor, areola, or the like, and contrast imaging can be performed by the above fluorescence observation device (such as a camera). In this case, the compound (A) becomes strong when it bonds with plasma protein in lymph fluid, and generates fluorescent light when irradiated with excitation light.

As described above, when using for angiography, sentinel lymph node mapping, or the like, the composition for fluorescent labeling is preferably liquid (25 degrees Celsius, relative humidity of 50%), from a viewpoint of preventing injection into body in an insoluble state. If the composition for fluorescent labeling is liquid, it may be possible to store it as a solution of high concentration (e.g., concentrated liquid) and to dilute it when using it. In this case, the dilution factor may be set so that the diluent has the above appropriate concentration and content. The concentrated liquid and the diluent can be prepared using a vial or the like of distilled water attached to the commercialized compound (A), for example.

In addition, as described later, the composition for fluorescent labeling can be used in clinic as an injection agent. The injection agent will be described later, and it may be liquid. In addition, the injection agent may be a gel-like or semi-solid state from a viewpoint of adding certain viscosity, shape retention, or the like. In order to make a gel-like or semi-solid state, it is sufficient to mix at least one component selected from a group consisting of (D) sodium alginate and sodium hyaluronate ((D) component), for example.

### ((C) Component: resin)

When using the composition for fluorescent labeling according to the present embodiment as various materials of a medical apparatus or the like, it is preferred that the composition for fluorescent labeling further includes resin (C). For instance, by using the composition for fluorescent labeling as molding material of a medical apparatus such as a stent, a tube, a catheter, a clip, a fluorescent coloring portion around a septum of a central venous port, a 3D printer filament, a resin fiber material, or the like, this medical apparatus can generate fluorescent light. Note that details of the medical apparatus will be described later.

Conventionally, it is considered that ICG is difficult to melt and knead with resin in many cases, because it has low heat resistance and is water-soluble. However, the inventor of this application found that the compound (A) can be melted and kneaded with the resin (C) to extent that enables use as molding material, by combining the above compound (A) and the alcoholic hydroxyl group-containing compound (B1).

Considering that ICG is water-soluble, the resin (C) is preferably a material that does not cause hydrolysis and has a low melting point lower than that of ICG (approximately 230 degrees Celsius). For instance, it preferably contain at least one selected from a group consisting of polyurethane resin, olefin resin, epoxy resin, vinyl chloride resin, fluorocarbon resin, polycarbonate resin, polyamide resin, ABS resin, acrylic resin, and silicone resin. These resins are readily available and have high stability, and hence are suitable as a molding material for a medical apparatus or the like. In addition, they are suitable also because they don't adversely affect fluorescent characteristics of the compound (A).

Polyurethane resin is a suitable resin. In this case, as the alcoholic hydroxyl group-containing compound (B1), it is preferred to use an organic solvent having a high boiling point (such as glycerol) rather than an organic solvent having a low boiling point, in order to effectively prevent elution of the compound (A) in conditions of high ambient temperature and humidity.

As olefin resin, there are polyethylene, polypropylene, and the like, for example.

The epoxy resin (B4) is expressed by the following structural formula.

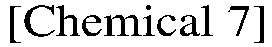

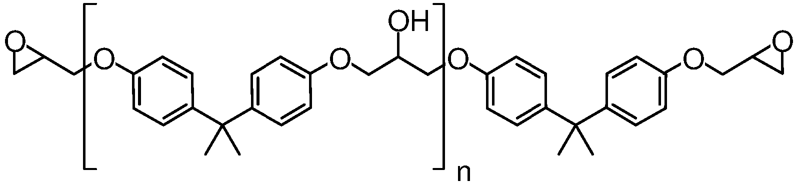

The epoxy resin (B4) does not always need the alcoholic hydroxyl group-containing compound (B1), because its hydroxyl group acts as a reinforcement factor of ICG. For this reason, compared with other resin, the epoxy resin (B4) has very strong fluorescence activity. As an example, there are epoxy resin in which bisphenol A, bisphenol F, novolac, or the like is glycidyl-etherified; epoxy resin in which propylene oxide, ethylene oxide, or polyalkylene glycol is added to bisphenol A to be glycidyl-etherified; aliphatic epoxy resin; alicyclic epoxy resin; polyether epoxy resin; and the like. In addition, the epoxy resin (B4) can be used for coating or the like of a metal, and it is suitable as a material attached to a tip of a various types of surgical appliances such as various forceps or scissors.

As the vinyl chloride resin, there are, for example, a homopolymer of vinyl chloride, a vinyl chloride-vinyl acetate copolymer, a vinyl chloride-ethylene copolymer, a vinyl chloride-propylene copolymer, a vinyl chloride-styrene copolymer, a vinyl chloride-vinylidene chloride copolymer, a vinyl chloride-acrylic ester copolymer, a vinyl chloride-ester maleate copolymer, a vinyl chloride-methacrylic acid ester copolymer, a vinyl chloride-acrylonitrile copolymer, and the like.

As the fluorocarbon resin, there are, for example, polytetrafluoroethylene, a tetrafluoroethylene-perfluoralkylvinyl ether copolymer, a tetrafluoroethylene-hexafluoropropylene copolymer, a tetrafluoroethylene-fluoro alkyl vinyl ether-fluoroolefin copolymer, an ethylene-tetrafluoroethylene copolymer, an ethylene-trichlorofluoroethylene copolymer, and the like.

As the polycarbonate resin, there are, for example, an aromatic polycarbonate resin containing bisphenol A and the like as copolymer compositions, an aliphaticpolycarbonate resin, an aromatic-aliphaticpolycarbonate resin, and the like.

As the polyamide resin, there are, for example, polytetramethylene adipamide (nylon 46), polytetramethylene sebacamide (nylon 410), polypentamethylene adipamide (nylon 56), polypentamethylene sebacamide (nylon 510), polycaproamide (nylon 6), polyhexamethylene adipamide (nylon 66), polyhexamethylene sebacamide (nylon 610), polyhexamethylene dodecamide (nylon 612), polydecamethylene adipamide (nylon 106), polydecamethylene sebacamide (nylon 1010), polydecamethylene dodecamide (nylon 1012), polyunde-caneamide (nylon 11), polyunde-camethylene adipamide (nylon 116), polydodecanamide (nylon 12), polyxylene adipamide (nylon XD6), polyxylene sebacamide (nylon XD10), polymethaxylene adipamide (nylon MXD6), polyparaxylylene adipamide (nylon PXD6), polytetramethyleneterephthalamide (nylon 4T), polypentamethylene terephthalamide (nylon 5T), polyhexamethylene terephthalamide (nylon 6T), polyhexamethylene isophthalamide (nylon 6I), polynonamethylene terephthalamide (nylon 9T), polydecamethylene terephthalamide (nylon 10T), polyundecamethylene terephthalamide (nylon 11T), polydodecamethylene terephthalamide (nylon 12T), polytetramethyleneisophthalamide (nylon 4I), polybis (3-methyl-4-aminohexyl) methanterephthalamide (nylon PACMT), polybis (3-methyl-4-aminohexyl) methanisophthalamide (nylon PACMI), polybis (3-methyl-4-aminohexyl) methandodecamide (nylon PACM12), polybis (3-methyl-4-aminohexyl) methan tetradecamide (nylon PACM14), and the like.

As the ABS resin, there are, for example, acrylonitrile butadiene styrene (ABS), acrylonitrile styrene acryl (ASA), acrylonitrile ethylene styrene (AES), polyethylene chloride acrylonitrile styrene (ACS), α-methylstyrene ABS resin, flame retardant ABS resin (FR-ABS), reinforced ABS resin, phenylmaleimide ABS resin, and the like.

As the acrylic resin, there are, for example, a homopolymer of polymethyl methacrylate (PMMA), polyethyl methacrylate, and the like; and a copolymer of methyl methacrylate-styrene copolymer, methyl methacrylate-α-methylstyrene copolymer, and the like.

As the silicone resin, there are, for example, methylsilicone resin, methylphenyl silicone resin, phenyl silicone resin, epoxy modified silicone resin, polyester modified silicone resin, acryl modified silicone resin, and the like.

Note that acetal resin may cause hydrolysis when mixed and kneaded with ICG, and can discharge hazardous substance such as formaldehyde. For this reason, it is preferred that the resin (C) does not contain acetal resin.

Only one type of the resin (C) may be used solely, or two or more types of the same may be combined when being used.

The content of the resin (C) in the composition for fluorescent labeling is not limited particularly, but is preferably 80 to 99.99998 wt%, more preferably 85 to 99.9999 wt%, and still more preferably 90 to 99.9997 wt%. By setting the content of the resin to be in the above range, the compartment identifiability and the light emission time by the fluorescent light emission can be maintained at high level, while strength, processing characteristics, deterioration resistance, and the like that are suitable for molding material can be obtained. Note that it was found from various experiment results that if the composition for fluorescent labeling contains the resin (C), there is a tendency that a high light emission intensity can be obtained when concentration of the compound (A) in the composition for fluorescent labeling is low to some extent. However, the characteristics vary depending on the resin.

When using the composition for fluorescent labeling as a molding material, the state of the composition for fluorescent labeling at 25 degrees Celsius and a relative humidity of 50% is preferably a solid or semisolid state. The semisolid state means being different from a liquid state and having shape retention like a paste state. If the composition for fluorescent labeling is in this state, handling ability, weighing accuracy, and uniform dispersibility in the molding material are improved, compared with the case where the compound (A) is directly kneaded. Further, when using the composition for fluorescent labeling as a molding material, it can be masterbatch.

As a method for realizing the solid or semisolid state of the composition for fluorescent labeling, the above resin (C) may be mixed, or the alcoholic hydroxyl group-containing compound (B1) in a solid or semisolid state may be mixed. In this case, as the above alcoholic hydroxyl group-containing compound (B1), glycerol or the like is suitable. In addition, other component in a solid or semisolid state may be mixed.

### <Method for Producing Composition for Fluorescent Labeling>

The method for producing the composition for fluorescent labeling of the present embodiment includes a mixing step of mixing the above compound (A) and the hydroxyl group-containing organic compound (B). As the hydroxyl group-containing organic compound (B), the above alcoholic hydroxyl group-containing compound (B1) can be used. The mixing method in the mixing step is not limited particularly, and can be selected from known methods. As mixing conditions in the mixing step, suitable conditions can be adopted appropriately.

In the mixing step, it is preferred to obtain the composition for fluorescent labeling containing the compound (A) of 0.0000001 to 10 wt% and the alcoholic hydroxyl group-containing compound (B1) of 1 to 90 wt%. The mixing step is not necessarily performed in one step. It may be possible to first perform the step of obtaining concentrated liquid at high concentration, and then to perform the step of diluting the concentrated liquid to be in the above concentration range when using it. In other words, dilute concentration when being used is preferably in the above concentration range.

As other steps, it may be possible to perform the step of heating the mixture (heating step) and the like after the mixing step, for example.

### <Fluorescent Probe>

The above composition for fluorescent labeling can be appropriately used as a fluorescent probe. According to the present embodiment, it can be used as an organic compound type fluorescent probe derived from the compound (A). For instance, when detecting lesion substance or physiologically active substance in a biological body, by putting the fluorescent probe inside the biological body or inside a cell, their behaviors can be visually identified and observed as fluorescence changes with high accuracy in real time. As described above, the composition for fluorescent labeling of the present embodiment does not allow the excitation light to be absorbed by hemoglobin or the like, and hence the excitation light reaches deep inside the biological body tissue, so that the state can be visually identified and observed. In addition, as to fluorescent light emission, it has good compartment identifiability, and secures a long life of the composition itself so as to continue to emit light for a long time. Because of such advantages, the composition for fluorescent labeling of the present embodiment is suitable as a fluorescent probe.

### <Injection Agent>

The above composition for fluorescent labeling can be appropriately used as an injection agent. A preferred embodiment of the injection agent of the present embodiment is an injection agent for labeling an object existing on mucous membrane or skin, and is an injection agent containing the above composition for fluorescent labeling. The above injection agent is injected to under the mucous membrane or skin on which the object is positioned, and the compound (A) is irradiated with the excitation light so that labeling is enabled.

The injection agent according to the present embodiment can be appropriately used in clinic. For instance, the injection agent is used for various types of local injection to under mucous membrane or skin of an excision target area such as a tumor. For instance, when performing a surgical procedure of removing a nidus such as a tumor formed on mucous membrane or skin, the injection agent is injected to under the mucous membrane or skin around the nidus, and is irradiated with the excitation light so that fluorescent light is generated. In this way, the transection line of the nidus (boundary between the remaining side and the removed side) can be accurately identified. In addition, by combining doctor's visual inspection view and the fluorescent observation, a more appropriate transection line can be determined.

In the case of the injection agent, the alcoholic hydroxyl group-containing compound (B1) as the hydroxyl group-containing organic compound (B) is preferably glycerol or mannitol in view of safety to the human body. One type of them may be used solely, or two or more types may be combined and used.

The injection agent according to the present embodiment can contain at least one of sodium alginate and sodium hyaluronate as the (D) component. This (D) component can also be allowed to function as a viscosity enhancer. Because the injection agent contains the (D) component, by injecting the injection agent to the under layer of mucous membrane or skin in a vicinity of the lesion, for example, the injection part can be expanded to rise. In this state, the above fluorescence observation device (such as a camera) is used for observation, the position of the nidus part can be accurately grasped, and the transection line of the nidus part can be identified more accurately.

Further, in the injection agent according to the present embodiment, the content of the (D) component is not limited particularly, but is preferably 0.01 to 10 wt% as the total of (D) component, more preferably 0.05 to 5 wt%, still more preferably 0.05 to 3 wt%, and still more preferably 0.1 to 1 wt%. However, if sufficient viscosity and locally limited property are obtained, it is effective even if the (D) component is not contained at all in the injection agent.

The content, combination, and the like of the above injection agent can also be applied to the above composition for fluorescent labeling, as a matter of course.

### <Syringe Filling>

The above composition for fluorescent labeling can be appropriately used as syringe filling. The syringe filling is constituted of the composition for fluorescent labeling of the present embodiment filled in a syringe (syringe barrel) in advance. The syringe filling is also referred to as a prefilled syringe or the like. The material, size, and shape of the syringe are not limited particularly, and appropriate conditions can be selected. For instance, the syringe may be made of glass, or may be made of resin. In addition, it may be possible to fill the composition for fluorescent labeling also in the tube of the local injection needle for injecting under the mucous membrane, and to connect the local injection needle to the prefilled syringe.

As to the composition for fluorescent labeling, it is important to appropriately adjust contents and content ratios of the compound (A), the hydroxyl group-containing organic compound (B), other solvent, and added ingredients, in order to suppress concentration quenching phenomenon, and to secure sufficient fluorescent light emission intensity and light emission time. However, it is complicated to mix these medicinal solutions at the time of operation. By filling the composition for fluorescent labeling, which is prepared in advance to have the accurate content ratio, into the syringe (plus the tube of the local injection needle), this complicated work can be eliminated at the time of operation. For instance, it is possible to fill the syringe with the concentrated liquid in advance, which contains the compound (A) and the alcoholic hydroxyl group-containing compound (B1) as the hydroxyl group-containing organic compound (B), at content ratios in the above appropriate ranges, and to dilute it for use with physiological sodium chloride solution, water for injection, or the like when it is used. Note that the syringe filling of the present embodiment is not limited to the configuration in which the concentrated liquid of the composition for fluorescent labeling is filled in the syringe in advance. For instance, it may be possible to fill the syringe with the composition for fluorescent labeling at a concentration (diluent) that can be injected as it is, so as to configure the syringe filling.

### <Medical Appliance>

The above composition for fluorescent labeling can be used as molding material for a medical apparatus. As such the medical apparatus, there are a stent, a tube, a catheter, a clip, a fluorescent coloring portion around a septum of a central venous port, a 3D printer filament, a resin fiber material (such as thread of gauze or surgical suture), and a various types of surgical appliances such as various forceps or scissors having a coated tip. Among these, the medical apparatus to which the composition for fluorescent labeling is applied is particularly and preferably one selected from a group consisting of a stent, a tube, a catheter, a fluorescent coloring portion around a septum of a central venous port, a 3D printer filament, a resin fiber material (such as medical gauze, sponge, tupfer gauze, swab, tampon, silk thread, or sponge), and a various types of surgical appliances such as various forceps or scissors.

For instance, if the stent is considered as the medical apparatus, the material constituting the stent is allowed to contain the composition for fluorescent labeling. The stent is positioned in a biological body, and the stent is irradiated with near-infrared rays as excitation light of the compound (A), so that the stent can generate fluorescent light. If the stent is a bile duct stent or an ureter stent, for example, the position of the stent can be accurately grasped by fluorescent light from the stent. In this way, it is possible to reduce risk of damaging the bile duct or the ureter by mistake during operation.

For instance, if the tube is considered as the medical apparatus, the material constituting the tube is allowed to contain the composition for fluorescent labeling. The tube is positioned in a biological body, and the tube is irradiated with near-infrared rays as excitation light of the compound (A), so that the tube can generate fluorescent light. For instance, in a case of an ileus tube, the fluorescent light enables to accurately grasp the position of the tube, and hence the area of ileus or the like can be identified more accurately even when laparoscopic surgery is performed.

For instance, if the catheter is considered as the medical apparatus, the material constituting the catheter is allowed to contain the composition for fluorescent labeling. The catheter is positioned in a biological body, and the catheter is irradiated with near-infrared rays as excitation light of the compound (A), so that the catheter generate fluorescent light. For instance, in a case of an ureter catheter, the fluorescent light enables to accurately grasp the position of the catheter, and hence it is possible to reduce risk of damaging the ureter by mistake during operation.

For instance, if the septum of a central venous port is considered as the medical apparatus, the central venous port, in which the composition for fluorescent labeling is contained in the material constituting the septum or a housing part as described in Patent Document 4, is not good for patient safety, because the resin containing medicine directly contacts with a subcutaneous tissue of the patient for a long time. The septum that is repeatedly punctured by the needle has a problem of coring that the material is gradually scraped, and containing the medicine is not good for fear of deterioration in durability, too. In addition, if the housing part contains medicine for fluorescent coloring, the light emission range becomes too wide, and the central septum part may not become fluorescent negative.

If the composition for fluorescent labeling is put inside the housing part around the septum (if it exists inner side of the outer surface of the housing part), it is possible to allow the position of the septum to be recognized while the light emission part does not contact with a subcutaneous tissue of the patient. The form of the composition for fluorescent labeling may be a ring shape or may be a set of spots, as long as it is disposed around the septum. Such the central venous port is disposed inside the biological body, and the above port is irradiated with near-infrared rays as excitation light of the compound (A), so that the composition for fluorescent labeling around the septum can generate fluorescent light.

In this way, even in a case of obese patient having thick subcutaneous fat, the position of the septum can be easily and accurately grasped to puncture, and mispuncture can be reduced. Therefore, it is possible to reduce risk of leakage of medicinal solution such as anticancer agent to a subcutaneous tissue by mistake due to mispuncture. In addition, it is also possible to minimize the port that has a tendency to be unnecessarily large to avoid mispuncture. As a result, cosmeticity of body wall of the patient can be improved. In addition, as to the septum and the housing part, the conventionally used material can be used as it is, except for the composition for fluorescent labeling, and hence barrier and cost for development can be minimized.

Further, by applying the composition for fluorescent labeling to a 3D printer filament, various types of "luminant medical apparatuses" can be easily manufactured for trial.

A method for producing the above medical apparatus according to the present embodiment is not limited particularly, but a known method can be adopted. For instance, it is possible to adopt a production method including the step of injection molding the above composition for fluorescent labeling with a die or the like. In addition, extrusion molding, compression molding, blow molding, calendar molding, inflation molding, thermoforming, or the like may be used for producing the medical apparatus.

In addition, for example, if the resin fiber material is considered as the medical apparatus, the above fiber material is allowed to contain the composition for fluorescent labeling. As the resin fiber material, as described above, for example, there are thread of gauze, surgical suture for operation, and the like. For instance, as to the gauze, as described in Patent Document 4, it is considered to weave the resin fiber material containing the composition for fluorescent labeling in a part of cotton cloth constituting the gauze, and to irradiate the above gauze with near-infrared rays as excitation light of the compound (A), so that the above fiber material constituting a part of the above gauze can generate fluorescent light. However, as only a part of the gauze generate light, it is considered that the light emission intensity is not sufficient for searching for it when it is missing or using it as indication for operation. On the other hand, if the mixing ratio of the resin fiber material is increased, the water absorbability is decreased, so that the original function of the gauze cannot be fulfilled. For this reason, it is preferred that the composition for fluorescent labeling is contained in the entire part of the cotton cloth constituting the gauze.

In addition, if the surgical suture contains the composition for fluorescent labeling, when irradiating the surgical suture with near-infrared rays as excitation light of the compound (A), the surgical suture generates fluorescent light. However, because the resin surgical suture is unabsorbable (is not absorbed in the body), the fiber containing the medicine contacts the patient tissue almost permanently, which is not good for patient safety. In addition, in the method of fluorescent coloring the needle, the needle is taken out of the body, but the resin is attached to the needle, and its original sharpness may deteriorated. Therefore, in a "suture with needle" that is a surgical suture whose tip is attached to a needle, for example, epoxy resin containing the compound (A) for example is used as adhesive connecting the needle and the suture, and thus it is possible to find a lost needle without the above problem.

According to the present embodiment, such the resin fiber material can be appropriately used as luminant fiber, luminant material, or the like in wide fields, without limiting to use in medical field (medical use). In addition, because the fiber material is a resin, the compound (A) can be uniformly dispersed and mixed in the resin, and hence the entire material can uniformly generate fluorescent light.

Note that, as a method for producing the resin fiber material, the composition for fluorescent labeling of the present embodiment (e.g., the compound (A) plus the hydroxyl group-containing organic compound (B)) may be mixed and kneaded with the resin for spinning, or only the compound (A) may be mixed and kneaded with the resin for spinning.

### <Medical Fiber Material>

The medical fiber material of the present embodiment contains the above compound (A) and the fiber material containing the hydroxyl group-containing organic compound (B), and can emit light when irradiated with the excitation light of the compound (A). When the above medical fiber material is irradiated with the excitation light of the compound (A), the medical fiber material generates fluorescent light, which has good compartment identifiability and a long light emission time. In order to facilitate and secure the development of the effect, the hydroxyl group-containing organic compound (B) contained in the fiber material preferably contains the cellulose (B2), and may further contain animal protein fiber such as silk (B3).

Here, a structural formula of the cellulose (B2) is shown below.

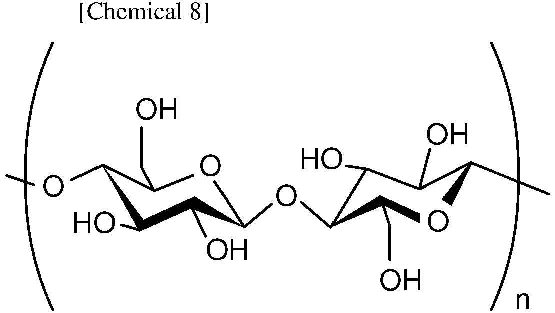

In addition, a structural formula of the silk (B3) is shown below.

[Chemical 9] G-A-G-A-G-S-G-A-A-G-(S-G-(A-G)*ₙ*)₈·T

Note that, in the above structural formulas, n = 2 holds. G represents glycin, A represents alanine, S represents serine, and T represents tyrosine.

A structural formula of serine is as follows.

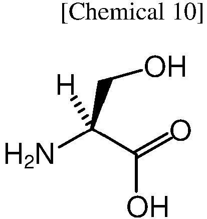

A structural formula of tyrosine is as follows.

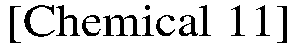

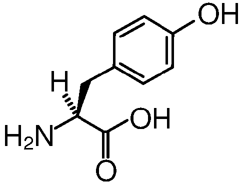

A specific example of this medical fiber material is not limited particularly, but there are, for example, medical gauze, sponge, tupfer gauze, swab, tampon, silk thread, and the like, or fiber material or the like constituting these material. For instance, if the medical fiber material according to the present embodiment is used as medical gauze, it can contribute to prevention of leaving gauze in a body in clinic, such as surgical operation. An example of use of this medical gauze is described below.

Conventionally, as a measure for preventing gauze from being left in a body, surgical gauze (X-ray gauze) or the like in which contrast thread is weaved is used, in which X-ray contrast agent is kneaded. If the X-ray gauze is left in a body, the contrast thread appears in the X-ray photograph, and the position of the gauze can be specified.

However, the contrast thread of the X-ray gauze appears as a thin and white object in the X-ray image, and is difficult to visually identify when it overlaps with bone, so that it may be overlooked even in the X-ray image. In this point, the medical gauze according to the present embodiment can be clearly viewed as the entire gauze by the fluorescent light in green or blue color, and hence the gauze can be easily checked by the simple method. In addition, because not the X-ray but the near-infrared light is used, radiation dose of the patient and staff can be reduced, resulting in high safety.

As the reason why the medical gauze according to the present embodiment can realize good compartment identifiability and a long light emission time, it is considered that the compound (A) and the hydroxyl group of the cellulose (B2) as the hydroxyl group-containing organic compound (B) contained in the gauze strongly bound with each other, by hydrogen bond action, so as to have a more stable form. (However, actions of the present embodiment are not limited to these.) Therefore, in a surgical operation, the gauze is held at an exfoliation target point in advance, and the fluorescent of this gauze is used as an index, so that the operation can be efficiently carried on.

It is sufficient that the gauze to be used contains the hydroxyl group-containing organic compound (B) (such as the cellulose (B2)), and other materials, size and shape, and the like are not limited particularly. For instance, the gauze to be used may be square-cut gauze that is used as surgical gauze (operation gauze) or the like, or may be cut gauze to be used for wound or stung place, folding gauze, wide gauze, or the like. In addition, the gauze may be sterilized by ethylene oxide gas (EOG), autoclave, or the like.

An additive amount of the compound (A) is not limited particularly, but it is preferably controlled to be a constant additive amount, in order to accurately grasp an amount of bleeding in the surgical operation, for example. For instance, the additive amount of the compound (A) per 1 m² of the gauze is preferably 0.001 to 10 g, more preferably 0.01 to 5 g, and still more preferably 0.1 to 3 g. In addition, even if blood attaches to the gauze, the fluorescent light is not blocked from being visually identified, and bonding between protein in blood and ICG increases light emission.

Note that the medical fiber material according to the present embodiment may further contain the above alcoholic hydroxyl group-containing compound (B1). In addition, the above medical fiber material may contain the other added ingredients including water described above, instead of the alcoholic hydroxyl group-containing compound (B1), or in addition to the alcoholic hydroxyl group-containing compound (B1).

The medical fiber material according to the present embodiment may further contain a mordant. Use of the mordant enables the compound (A) together with dye to fix to gauze. As the mordant, alum, iron oxide, or the like can be used.

Although the example of the case where the medical fiber material according to the present embodiment is used as the medical gauze is described above, the medical fiber material according to the present embodiment is not limited to this use. For instance, also when using the medical fiber material as sponge, tupfer gauze, swab, tampon, silk thread (with a needle), or the like, or a fiber material constituting the same, fluorescent light can be generated when using it, similarly to the medical gauze. Note that the above silk thread (with a needle) may be used as a target in the second stage surgery, for example. When the silk thread (with a needle) generates fluorescent light, the above target can be easily recognized, which is very useful when performing the second stage surgery.

### <Method for Producing Medical Fiber Material>

A method for producing medical fiber material according to the present embodiment includes an impregnation step and a mordanting step. In the impregnation step, the fiber material containing the hydroxyl group-containing organic compound (B) is impregnated with solution containing the above compound (A). In the mordanting step, the compound (A) is fixed to the fiber material using the mordant.

The fiber material is not limited particularly, but it is sufficient if it contains the hydroxyl group-containing organic compound (B) and can be impregnated with the solution containing the compound (A). In this case, the hydroxyl group-containing organic compound (B) preferably contains the cellulose (B2) or the silk (B3). Note that the silk (B3) is a protein having the hydroxyl group-containing amino acid as a component, as described above. As this fiber material, there are medical gauze, sponge, tupfer gauze, swab, tampon, silk thread (with a needle), and the like, or a fiber material or the like constituting the same, for example. For instance, the medical gauze, sponge, tupfer gauze, swab, tampon, silk thread (with a needle), or the like may be directly impregnated with the above solution, or the fiber material constituting the medical gauze, sponge, tupfer gauze, swab, tampon, silk thread (with a needle) or the like may be impregnated with the above solution. In addition, other than the other fiber material described in this specification, a fiber material or the like that is generally used in the medical field or other field can also be used. Note that the above impregnation step may be performed by spraying. In other words, the impregnation means that the fiber material is impregnated with the above solution in this description, and the fiber material may be dipped in the above solution for impregnation, or the above solution may be sprayed to the fiber material for impregnation.

For instance, when the fiber material such as gauze is impregnated with the above solution, in the impregnation step, the fiber material may be impregnated with the solution containing at least the compound (A) and the alcoholic hydroxyl group-containing compound (B1) as the hydroxyl group-containing organic compound (B). In this case, the alcoholic hydroxyl group-containing compound (B1) can be used as solvent of the compound (A). Impregnation conditions are not limited particularly, and suitable conditions can be appropriately selected in consideration of the material, size, shape, and the like of the fiber material. For instance, impregnation time is preferably 1 minute to three days under normal temperature and humidity, and it is preferred to shield light when impregnation is performed. Note that the impregnation may be performed by spraying as described above.

The solution that is used in the method for producing medical fiber material may be composition containing the compound (A), the alcoholic hydroxyl group-containing compound (B1), and water. Note that the above solution is not always required to contain the alcoholic hydroxyl group-containing compound (B1). In other words, the above solution may contain only the compound (A) and water.

When using the alcoholic hydroxyl group-containing compound (B1), it is possible to use, for example, an alcoholic hydroxyl group-containing compound having a low boiling point or volatility, such as methanol, ethanol, isopropyl alcohol, or the like, or an alcoholic hydroxyl group-containing compound having a high boiling point, such as glycerol.

If the alcoholic hydroxyl group-containing compound having a low boiling point or volatility is used as the alcoholic hydroxyl group-containing compound (B1), when the gauze is impregnated with the composition for fluorescent labeling, the compound (A) is supported on the gauze. After that, the alcoholic hydroxyl group-containing compound (B1) is volatilized or removed so that the compound (A) is supported on the gauze.

If the alcoholic hydroxyl group-containing compound having a high boiling point is used as the alcoholic hydroxyl group-containing compound (B1), after the gauze is impregnated with the above solution so that the compound (A) is supported on the gauze, the alcoholic hydroxyl group-containing compound (B1) is not removed but is supported on the gauze together with the compound (A).

In the present embodiment, the above alcoholic hydroxyl group-containing compound (B1) is preferably the alcoholic hydroxyl group-containing compound having a low boiling point or volatility, more preferably ethanol or isopropyl alcohol, and still more preferably ethanol. By using this component as the alcoholic hydroxyl group-containing compound (B1), with the simple method, good economic efficiency is obtained, and only the compound (A) is supported on the medical gauze.

In the present embodiment, as the mordanting step using the mordant, the step of dyeing the gauze with the compound (A) using the mordant is performed, for example. The dyeing step may be pre-mordanting, or may be post-mordanting, or may be simultaneous mordanting. In order to dye the gauze with the compound (A), it is preferably the post-mordanting. As the mordant, one described above can be used. The mordanting conditions are not limited particularly.

Before dyeing using the mordant, it may be possible to perform a protein processing step of allowing the fiber material to be impregnated with a protein product. The protein processing enables the dye and the compound (A) to fix to the gauze. As the above protein product, it is possible to use milk, soy milk, skim milk powder, or the like.

Further, in the present embodiment, it is preferred to perform a sterilization step as necessary. The sterilization conditions are not limited particularly, and a known method can be adopted as the sterilization method of the medical fiber material such as medical gauze.

It may be possible to make an appliance by coating a tip or the like of a surgical appliance such as various forceps or scissors with epoxy resin containing the composition for fluorescent labeling, for example. In this case, operation can be performed while visually checking the tip or the like of the appliance that is hidden behind an organ or the like, and hence safety in operation can be improved.

As described above, the composition for fluorescent labeling according to the present embodiment has at least good compartment identifiability to extent that enables the structure or the like of the observation target to be identified more accurately, and has a long light emission time, so that the observation target can be observed for a long time. Further, a load to a biological body is small, and economic efficiency is good.

In particular, in the biological fluorescence imaging using the composition for fluorescent labeling according to the present embodiment, the compound (A) that is the near-infrared fluorescent organic dye is used, and hence the near-infrared light having a longer wavelength than the visible light is used as the excitation light. For this reason, in the biological fluorescence imaging, it is not necessary to detect fluorescent light from substances contained in the biological body tissue (self-fluorescent light), and there are little noises, which is an advantage. Further, by combining with the hydroxyl group-containing organic compound (B) (in particular, the alcoholic hydroxyl group-containing compound (B1)), stable fluorescent coloring and sufficient fluorescence intensity can be obtained, unlike the conventional method.

In addition, the excitation light is not absorbed by a substance in the biological body tissue such as hemoglobin, so that the light is allowed to reach deep inside the biological body tissue, and there is also an advantage that a state of blood vessel, lymph vessel, organ, or the like can also be observed.

This composition for fluorescent labeling is useful also as a fluorescent probe, an injection agent, a syringe filling, a medical apparatus, a medical fiber material, or the like, and the above advantage can be obtained. In addition, in the method for producing the composition for fluorescent labeling or the medical fiber material, a readily available material or component can be used, and the production can be performed by the easy method. Thus, there is also an advantage as the production method.

According to the present embodiment, the composition for fluorescent labeling, the fluorescent probe, the injection agent, the syringe filling, the medical apparatus, or the medical fiber material has the above various advantages, and hence can be appropriately used for the biological fluorescence imaging using the ICG compound (compound (A)). For instance, in a medical front, it can be applied to angiography, tumor fluorescence imaging, regional lymph node mapping including sentinel lymph node mapping, labeling of a surgical operation target, labeling of an organ whose damage should be avoided, prevention of leaving in a body, prevention of missing, and the like. Further, other than the medical front, it is also expected to be applied to fields such as basic medical research, life science, regeneration medicine, various diagnoses, architectural engineering, and entertainment. In other words, the composition or the like for fluorescent labeling of the present embodiment is not limited to medical use but can be expected to be widely applied to other various uses.

For instance, in a building, if a builder wants to label an architectural point or place that a user (owner) does not need to know, the composition for fluorescent labeling described above in the present embodiment is mixed and kneaded with or applied on a building material, and thus the builder can grasp the specific place (the place where the composition for fluorescent labeling exists) without allowing the user to know. In addition, if the composition for fluorescent labeling described above in the present embodiment is used to make a "luminant contact lens", a performer who wears the "luminant contact lens" on the eye does not feel the fluorescent light, but viewers can recognize the eye's light (light emission of the contact lens). In other words, the composition for fluorescent labeling of the present embodiment can be expected to be applied to a performance of a play.

### <Verification>

### [Mixing and Kneading of ICG and Resin]

Pigment (ICG plus solvent) was mixed and kneaded with various types of resin, and then it was verified whether or not a sheet could be manufactured by compression molding.

As the various types of resin, five types were prepared, which include polypropylene(PP), polyvinyl chloride (PVC), low-density polyethylene(LDPE), ethylene-vinyl acetate copolymer resin (EVA), and thermoplastic polyurethane (TPU). As the pigment, viscous liquid was used in which four 25 mg of ICG were mixed and dissolved with 10 ml of glycerol.

In addition, four patterns, i.e., 1 phr, 3 phr, 5 phr, and 7 phr of the above pigment were prepared. Note that phr is abbreviation of per hundred resin, which is a unit expressing a ratio of pigment mixed in resin whose weight is 100. For instance, if A g of pigment is in 100 g of resin, the pigment ratio can be said to be A (phr).

A procedure of mixing and kneading was as follows.

### (Step 1: Preliminary Mixing and Kneading)

The resin was charged into a mixer that had been preheated, and was kneaded until it was plasticized. As the above mixer, Laboplast mill 4 M150 manufactured by Toyo Seiki Seisaku-sho, Ltd. was used. Note that "Laboplast mill" is a registered trademark of Toyo Seiki Seisaku-sho, Ltd. This step was performed for each resin prepared in advance. Mixing and kneading was performed for five minutes at temperature set for each resin. The above mixing and kneading temperature was specifically 170 degrees Celsius for polypropylene, 150 degrees Celsius for polyvinyl chloride, 140 degrees Celsius for low-density polyethylene, 140 degrees Celsius for ethylene-vinyl acetate copolymer resin, and 150 degrees Celsius for thermoplastic polyurethane. Note that each mixing and kneading temperature is lower than the melting point of ICG (230 degrees Celsius).

### (Step 2: Pigment Mixing and Kneading)

After confirming that the resin was sufficiently plasticized in Step 1, the pigment was charged, mixed and kneaded. The mixing and kneading conditions (time and temperature) were the same as in Step 1. After that, the mixed and kneaded sample (resin plus pigment) was recovered.

### (Step 3: Compression Molding)

The above sample was finely cut by scissors. Then, the cut sample was sandwiched between a pair of polyimide films having heat resistance, and this sample covered with film was placed between a pair of metal plates (e.g., stainless steel plates). Next, the pair of metal plates including the sample covered with film is placed between a pair of spacers. After that, the pair of spacers were pressed in the direction of approaching each other, and the sample was melted and pressed for one minutes until being a desired thickness, so as to form a sheet. Compression molding temperature was the same as in Step 1 or 2. After molding, the pair of metal plates was placed on a mounting table, and was cooled at room temperature in a state where a weight was placed on the same. Note that the above thickness of the sheet was obtained in two values, i.e., 1 mm (the area of 110 mm × 110 mm) and 0.5 mm (the area of 125 mm × 125 mm).

### (Results)

It was confirmed that kneading of pigment with resin was able to be performed without a problem for every resin. In addition, it was confirmed that kneading was able to be performed without a problem for any ratio of the pigment. In this way, it can be concluded that thermal degradation of ICG due to mixing and kneading can be suppressed.

### [Verification of Gauze]

Next, the gauze was impregnated with the solution containing ICG, then the gauze was irradiated with near-infrared light, and it was verified whether or not the gauze was able to generate fluorescent light. The process and results of the verification are described below.

### (Preparation of Undiluted Solution)

First, as the undiluted solution, the G solution, the E solution, the N solution, and the A solution, and distilled water were prepared. The G solution is a solution in which 25 mg of ICG is mixed and dissolved with 10 ml of glycerol. The E solution is a solution in which 25 mg of ICG is mixed and dissolved with 10 ml of ethanol. The N solution is a solution in which 25 mg of ICG is mixed and dissolved with 10 ml of distilled water. The A solution is a solution in which 25 mg of ICG is mixed and dissolved with 10 ml of 5% BSA solution. Note that BSA is bovine serum albumin. The distilled water, which did not contain ICG, was prepared for comparison with the undiluted solutions containing ICG.

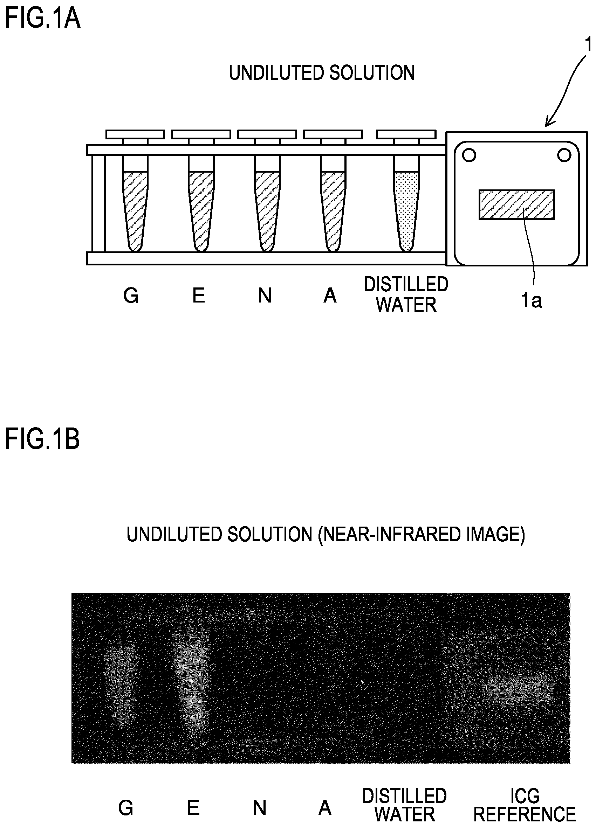

FIG. 1A schematically illustrates a state where the undiluted solutions in containers (transparent containers) are arranged. In FIG. 1A, the containers containing respectively the G solution, the E solution, the N solution, the A solution, and the distilled water are arranged in this order from the left. An ICG reference card 1 is placed on the right side of the container containing the distilled water. The ICG reference card 1 has a light emission area 1a. The light emission area 1a is an area containing ICG, and generates fluorescent light when irradiated with excitation light (near-infrared light). Here, with reference to light emission luminance of the light emission area 1a of the ICG reference card 1, presence or absence of light emission when irradiated with near-infrared light and luminance of the light emission part are relatively compared and evaluated. In other words, the ICG reference card 1 is used for relative comparison and evaluation of light emission luminance.

FIG. 1B illustrates an image (hereinafter, referred to also as a near-infrared image) obtained by irradiating the containers with near-infrared light, and by imaging the containers with a near-infrared light camera (VISERAELITE II manufactured by Olympus Corporation). From this illustration, it is understood that the G solution and the E solution have high light emission luminance in the undiluted solution state.

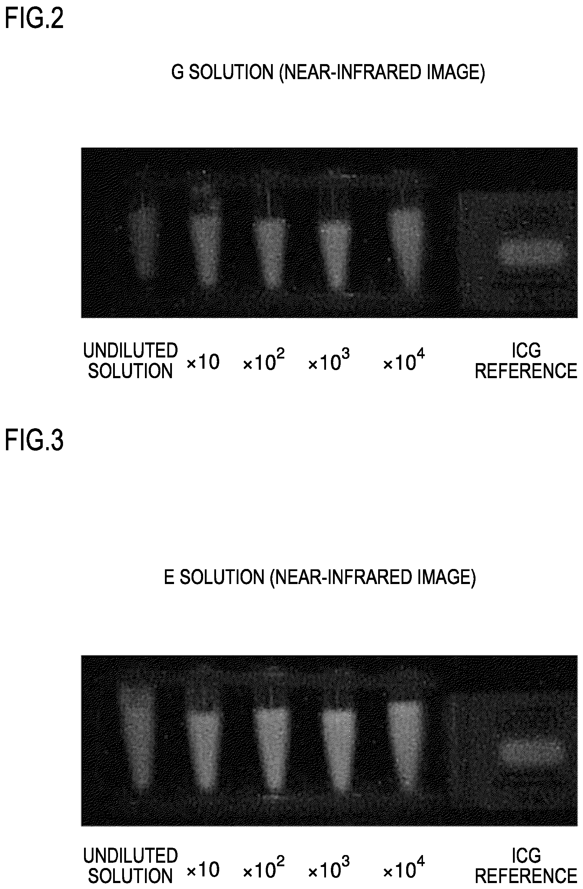

Next, each undiluted solution was diluted by 5 times, 10 times (×10¹), 100 times (×10²), 1,000 times (×10³), and 10,000 times (×10⁴) so as to produce diluents. Note that solvent of each undiluted solution was used for dilution. FIG. 2 illustrates a near-infrared image obtained by the G solution. Note that in FIG. 2, the container containing the 5 times dilution solution is omitted (the same is true in FIGS. 3 to 5). It is understood from FIG. 2 that good (high) light emission luminance can be obtained by the G solution of any dilution factor.

FIG. 3 illustrates a near-infrared image obtained by the E solution. From this illustration, it is understood that good (high) light emission luminance can be also obtained by the E solution of any dilution factor, similarly to the G solution.

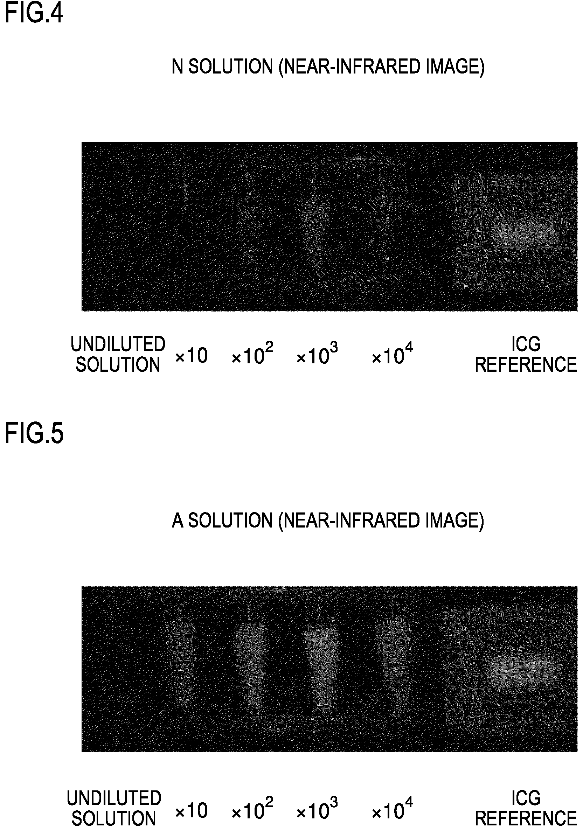

FIG. 4 illustrates a near-infrared image obtained by the N solution. From this illustration, it is understood that high light emission luminance can be obtained by the N solution of 1,000 times dilution, and that the light emission luminance is low at other dilution factors.

FIG. 5 illustrates a near-infrared image obtained by the A solution. From this illustration, it is understood that high light emission luminance can be obtained by the A solution of 10 times dilution to 10,000 times dilution, and that higher light emission luminance can be obtained at 100 times dilution and 1,000 times dilution in particular.

### (Impregnation of Gauze)

Next, a diluent of a desired dilution factor was selected from the above prepared diluents (here, distilled water was used, but solvent of each undiluted solution may be used), and the gauze was impregnated with the selected diluent, so as to acquire the near-infrared image. Here, as the gauze, Sterase manufactured by Hakujuji Co., Ltd. (sterilized, 5 cm × 5 cm) was used. Note that "Sterase" is a registered trademark of Hakujuji Co., Ltd.

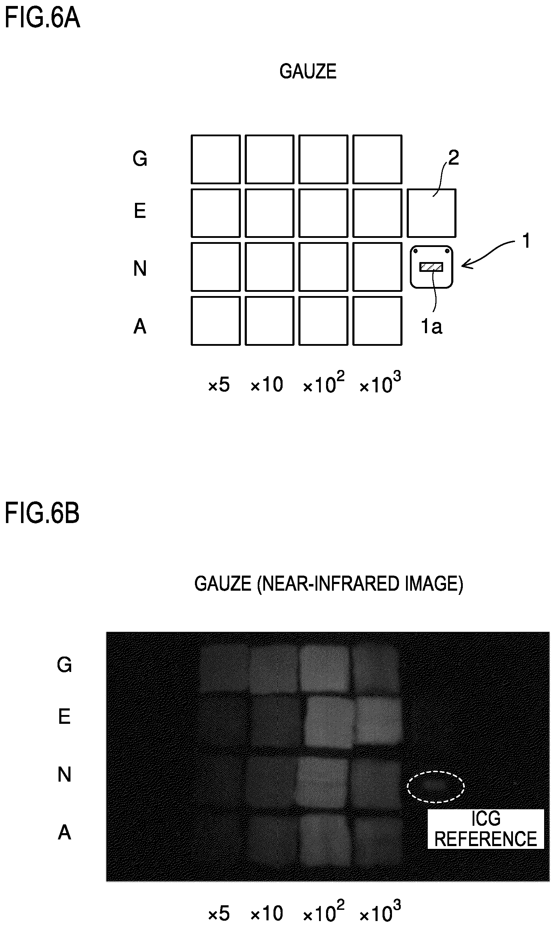

FIG. 6A schematically illustrates a state where gauzes are arranged lengthwise and crosswise, which are respectively impregnated with diluents of the G solution, the E solution, the N solution, and the A solution, diluted by 5 times, 10 times, 100 times, and 1,000 times for each solution. In FIG. 6A, from top to bottom, the gauzes are impregnated with diluents of the G solution, the E solution, the N solution, and the A solution, while from left to right, the gauzes are impregnated with diluents diluted by 5 times, 10 times, 100 times, and 1,000 times. In addition, in FIG. 6A, the ICG reference card 1 is placed on the right side of the gauze impregnated with the diluent diluted by 1,000 times, and an untreated gauze 2 is placed on the upper side of the ICG reference card 1. The untreated gauze 2 is a gauze that is not impregnated.

FIG. 6B illustrates a near-infrared image obtained when the gauzes of FIG. 6A are irradiated with near-infrared light. From this illustration, it was found that high light emission luminance was able to be obtained by each of the gauzes impregnated with the diluents of the G solution, the E solution, the N solution, and the A solution, diluted by 10 times to 1,000 times for each solution. In particular, it was found that the maximum light emission luminance was able to be obtained by the gauze impregnated with the diluent diluted by 100 times.

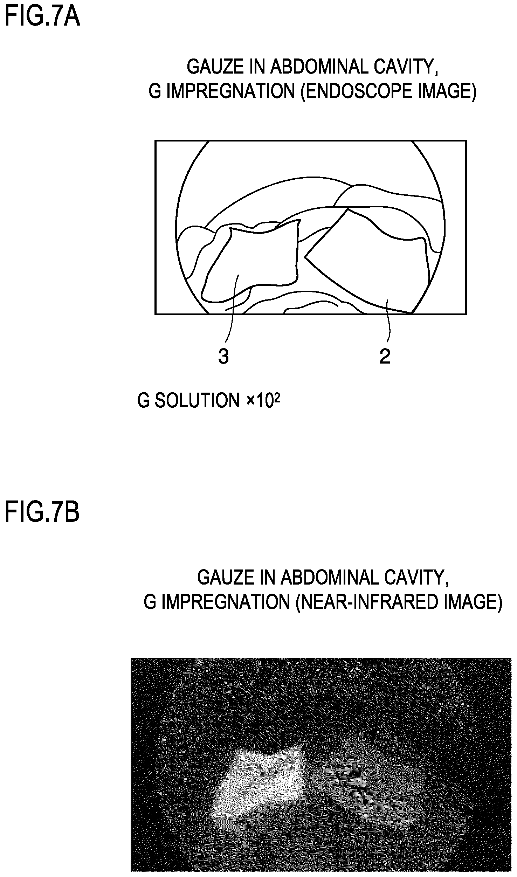

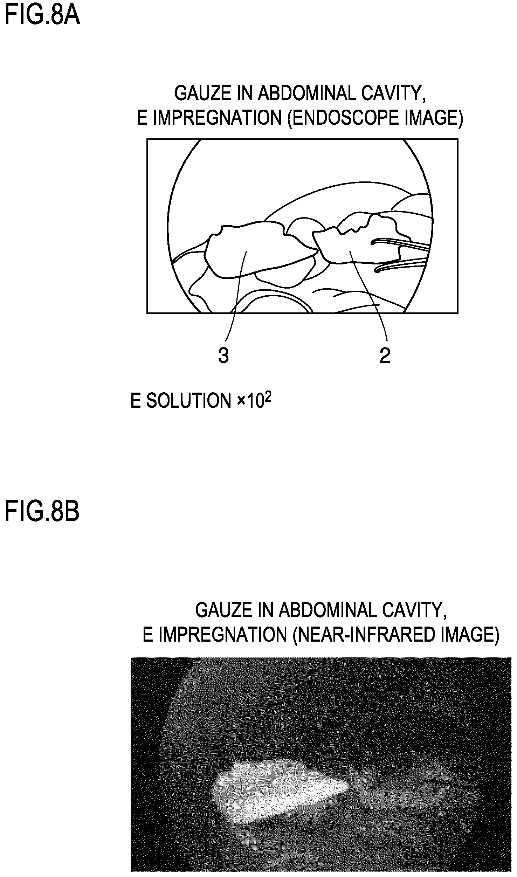

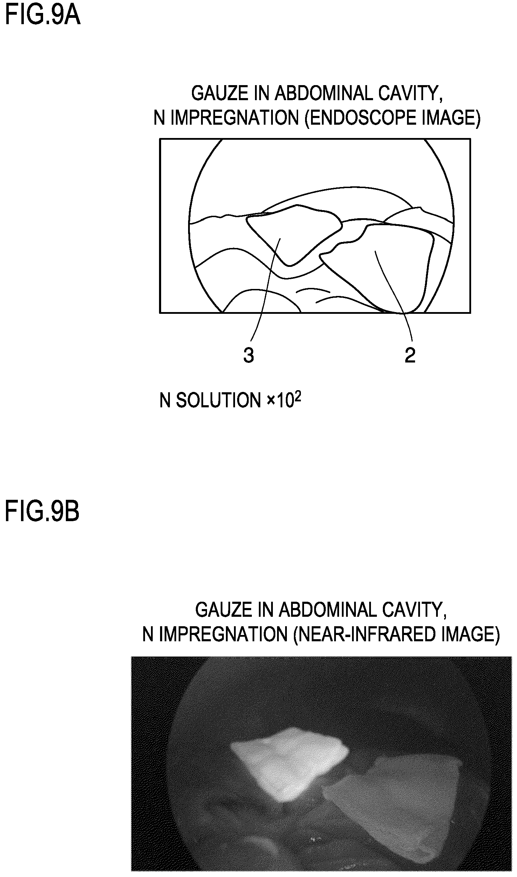

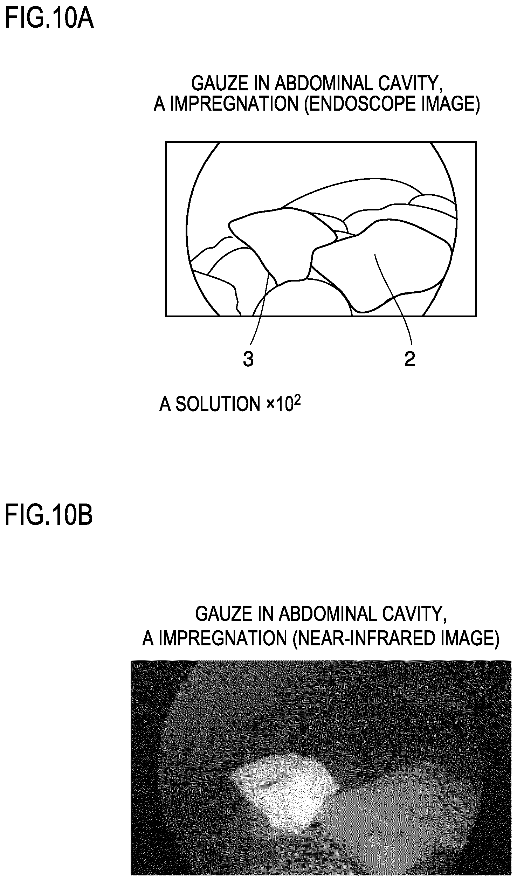

Next, results of animal test using the above gauzes are described. FIGS. 7A, 8A, 9A, and 10A schematically illustrate images taken by placing the untreated gauze 2 and a treated gauze 3 in a pig's abdominal cavity, and by using an endoscopic camera. Here, as the treated gauze 3 of FIG. 7A, the gauze impregnated with diluent of the G solution diluted by 100 times was used. As the treated gauze 3 of FIG. 8A, the gauze impregnated with diluent of the E solution diluted by 100 times was used. As the treated gauze 3 of FIG. 9A, the gauze impregnated with diluent of the N solution diluted by 100 times was used. As the treated gauze 3 of FIG. 10A, the gauze impregnated with diluent of the A solution diluted by 100 times was used.

FIGS. 7B, 8B, 9B, and 10B illustrate near-infrared images obtained by irradiating the gauzes of FIGS. 7A, 8A, 9A, and 10A with near-infrared light. From these illustrations, it was found that the treated gauzes 3 generated high intensity light in the abdominal cavity when irradiated with near-infrared light.

### [Structural Example of Central Venous Port]

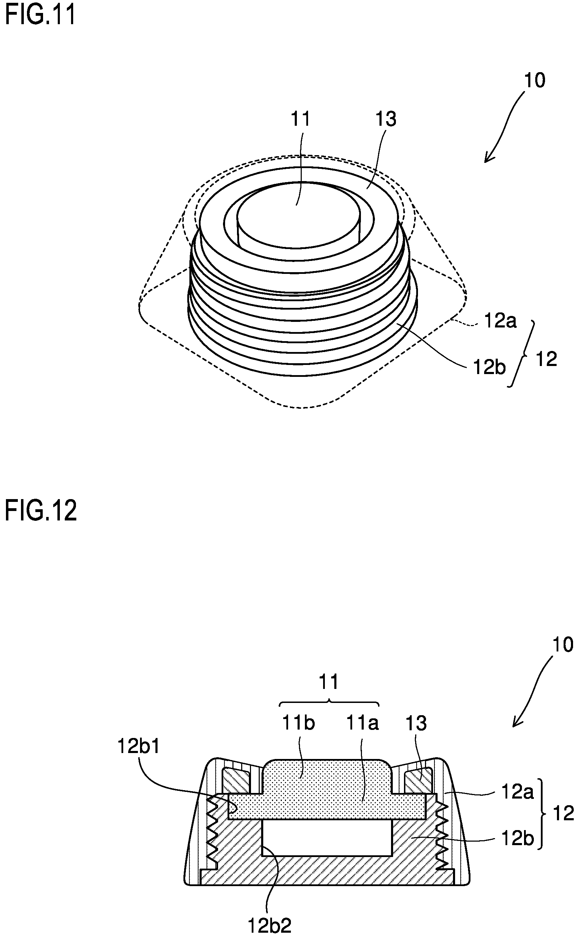

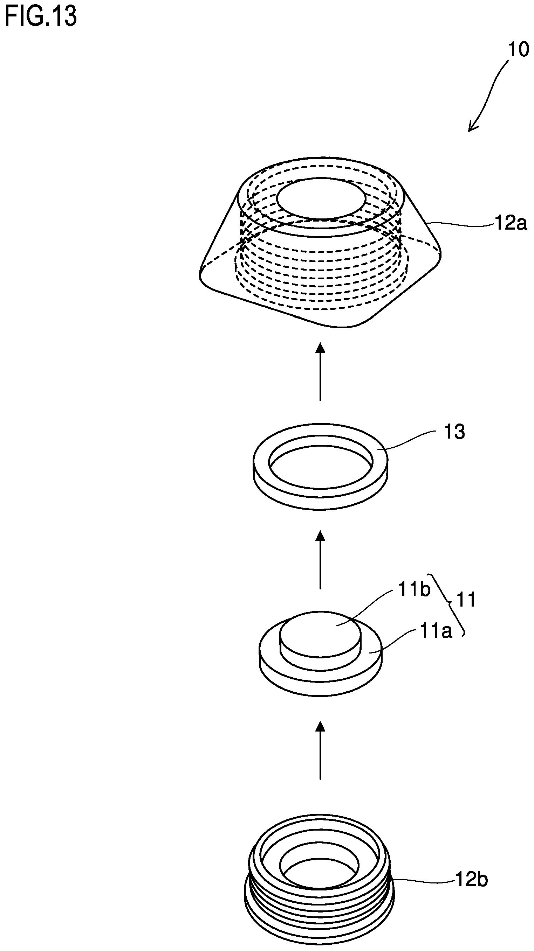

FIG. 11 is a perspective view illustrating a structural example of a CV port (central venous port) 10. FIG. 12 is a cross-sectional view of the CV port 10 of FIG. 10. FIG. 13 is an exploded perspective view of the CV port 10 of FIG. 10. Note that in FIG. 11, a contour of a housing part 12 is shown by a broken line in order to clarify an internal structure of the CV port 10.

The CV port 10 includes a septum 11, the housing part 12, and an index member 13. The septum 11 is made of soft silicone rubber, for example. The septum 11 integrally has a large diameter part 11a and a small diameter part 11b. The small diameter part 11b has a smaller diameter than the large diameter part 11a. The small diameter part 11b is positioned coaxially with the large diameter part 11a on the upper side of the large diameter part 11a. A Huber needle for injecting medicinal solution is inserted into the septum 11 (e.g., the small diameter part 11b).

The housing part 12 is a cover for holding the septum 11, and holds the septum 11 in such a manner that the upper side of the septum 11 (e.g., the small diameter part 11b) is exposed. Such the housing part 12 includes an outer housing 12a and an inner housing 12b.

The outer housing 12a is made of transparent acrylic resin, for example. When the CV port 10 is embedded under skin of a patient, a part of the outer surface (e.g., the upper surface) of the outer housing 12a contacts the skin of the patient. A thread groove is formed on the inner circumferential surface of the outer housing 12a. The inner housing 12b is a back cover positioned inside of the outer housing 12a. A screw thread is formed on the outer circumference surface of the inner housing 12b. Allowing the above screw thread and the above thread groove to engage with each other, the inner housing 12b is screwed into the outer housing 12a, and hence the inner housing 12b is fixed to the outer housing 12a.

A recess 12b1 is formed on the upper part of the inner housing 12b. The large diameter part 11a of the septum 11 is engaged with the recess 12b1 and is sandwiched vertically between the inner housing 12b and the outer housing 12a.

A liquid injection chamber 12b2 for containing the above medicinal solution is formed in the inner housing 12b. The liquid injection chamber 12b2 is formed to have a smaller diameter than the recess 12b1, and is positioned at the lower part of the recess 12b1. The medicinal solution injected into the liquid injection chamber 12b2 via the Huber needle inserted in the septum 11 is introduced to a blood vessel or the like, via a catheter (not shown) communicated to the liquid injection chamber 12b2.

The index member 13 is positioned inside the housing part 12 and outside the septum 11. More specifically, the index member 13 is positioned at the inner side of the contour of the housing part 12 (particularly the outer housing 12a). In addition, the index member 13 is positioned on the upper surface of the large diameter part 11a of the septum 11, apart from the small diameter part 11b in the radial direction (see particularly FIG. 12). The index member 13 is covered with the outer housing 12a. Therefore, a part of the outer housing 12a is positioned between the index member 13 and the small diameter part 11b.

The index member 13 is a ring-like member surrounding the septum 11 (particularly the small diameter part 11b). Note that the shape of the index member 13 is not limited to a ring (circle in a plan view), but may be other shapes such as a square or an ellipse. In addition, the index member 13 is not necessarily formed continuously in the circumferential direction, but a plurality of the index members may be disposed with spaces in the circumferential direction, for example.

The index member 13 contains the above composition for fluorescent labeling of the present embodiment. Specifically, the index member 13 is made by mixing and kneading the above pigment (ICG plus solvent) as the composition for fluorescent labeling with the resin, and by molding the mixed and kneaded resin in a ring-like shape, for example. In the structure of the index member 13 containing the composition for fluorescent labeling, when irradiated with near-infrared light, not the septum 11 itself but the part (the index member 13) inside the housing part 12, which does not directly contact with a subcutaneous tissue of the patient, is allowed to emit fluorescent light, so that the septum 11 can be recognized. For this reason, the problem that the resin containing the medicine directly contacts with a subcutaneous tissue of the patient for a long time does not occur, and safety can be improved for sure.

In particular, because the index member 13 has a shape (e.g., a ring-like shape) surrounding the septum 11 in a plan view, it is easily recognized that the septum 11 exists in the region surrounded by the index member 13. For this reason, the Huber needle can be easily inserted using the above region as an indicator.

### [Verification of Light Emission Intensity of Index Member]

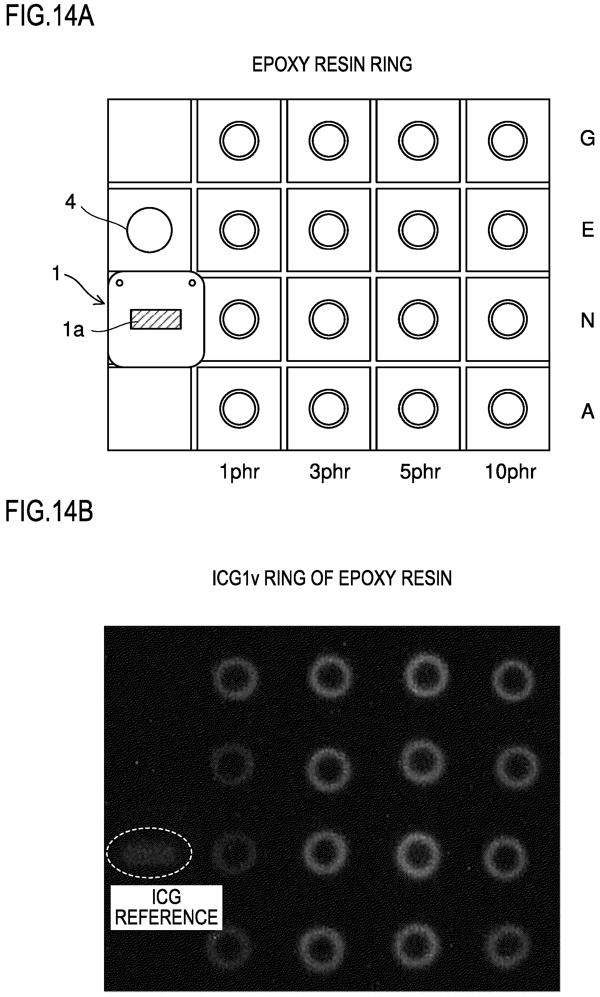

Next, results of verification of light emission intensity of the index member 13 are described below. FIG. 14A schematically illustrates a state where rings (corresponding to the index members 13) are arranged lengthwise and crosswise, the rings being molded by mixing the G solution, the E solution, the N solution, and the A solution, respectively, with epoxy resin at ratios of 1 phr, 3 phr, 5 phr, and 10 phr. In FIG. 14A, from top to bottom, the rings molded by mixing the G solution, the E solution, the N solution, and the A solution, respectively, with epoxy resin are arranged in this order. In addition, in FIG. 14A, the ICG reference card 1 is disposed at the left end, and a negative control 4 is disposed on the upper side of the ICG reference card 1. The negative control 4 is used for comparison with the rings and is made of epoxy resin that does not contain ICG. Further in FIG. 14A, on the right side of the ICG reference card 1 and the negative control 4, from left to right, the rings of the ratios of 1 phr, 3 phr, 5 phr, and 10 phr are arranged in this order.

FIG. 14B illustrates a near-infrared image obtained by irradiating the rings (ICG1v rings) of FIG. 14A with near-infrared light. Note that the ICG1v rings are rings molded by mixing the G solution, the E solution, the N solution, and the A solution with epoxy resin as follows. In other words, the G solution is a solution in which 25 mg of ICG is mixed and dissolved with 10 ml of glycerol. The E solution is a solution in which 25 mg of ICG is mixed and dissolved with 10 ml of ethanol. The N solution is a solution in which 25 mg of ICG is mixed and dissolved with 10 ml of distilled water. The A solution is a solution in which 25 mg of ICG is mixed and dissolved with 10 ml of 5% BSA solution. From this illustration, it was found that light emission luminance that is the same as or more than that of the ICG reference card was able to be obtained by the ring using any one of the G solution, the E solution, the N solution, and the A solution. In particular, it was found that higher light emission luminance was able to be obtained by the ring of 3 phr or more.

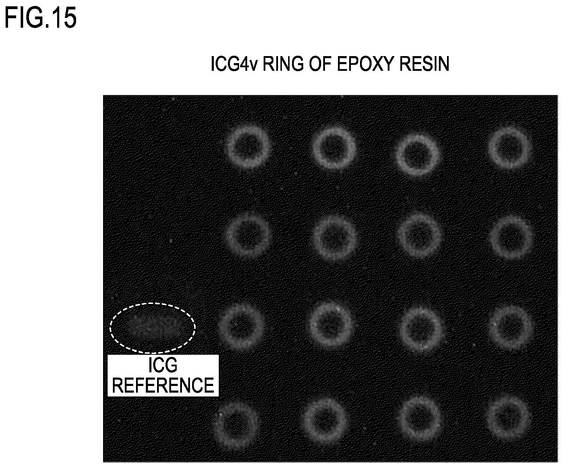

FIG. 15 illustrates a near-infrared image obtained by replacing the rings (ICG1v rings) of FIG. 14A with ICG4v rings, and by irradiating the rings with near-infrared light. Note that the ICG4v rings are rings molded by mixing the G solution, the E solution, the N solution, and the A solution with epoxy resin as follows. In other words, the G solution is a solution in which 100 mg of ICG is mixed and dissolved with 10 ml of glycerol. The E solution is a solution in which 100 mg of ICG is mixed and dissolved with 10 ml of ethanol. The N solution is a solution in which 100 mg of ICG is mixed and dissolved with 10 ml of distilled water. The A solution is a solution in which 100 mg of ICG is mixed and dissolved with 10 ml of 5% BSA solution. It was found that higher light emission luminance was able to be obtained by the ICG4v rings, i.e., all the rings of 1 phr to 10 phr, molded using any one of the G solution, the E solution, the N solution, and the A solution.

Note that, when grinding the surface of each ring, there was a tendency that higher light emission intensity was able to be obtained in the near-infrared image.

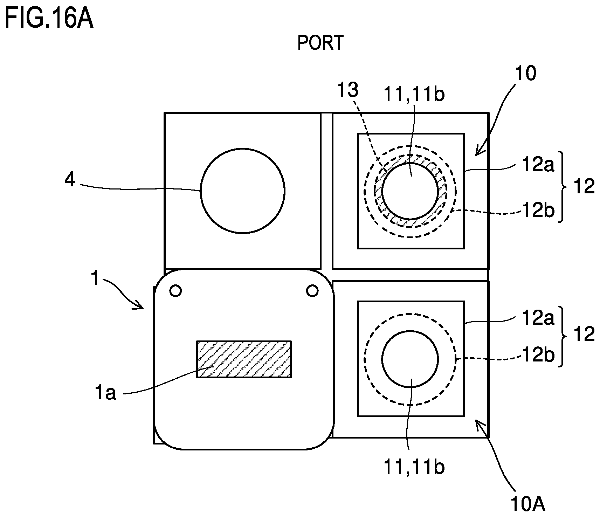

FIG. 16A schematically illustrates a state where the CV port 10 including the index member 13 and a comparison port 10A that does not include the index member 13 are arranged up and down. In FIG. 16A, the negative control 4 is disposed on the left side of the CV port 10. Further, the ICG reference card 1 is disposed on the lower side of the negative control 4 (on the left side of the comparison port 10A).

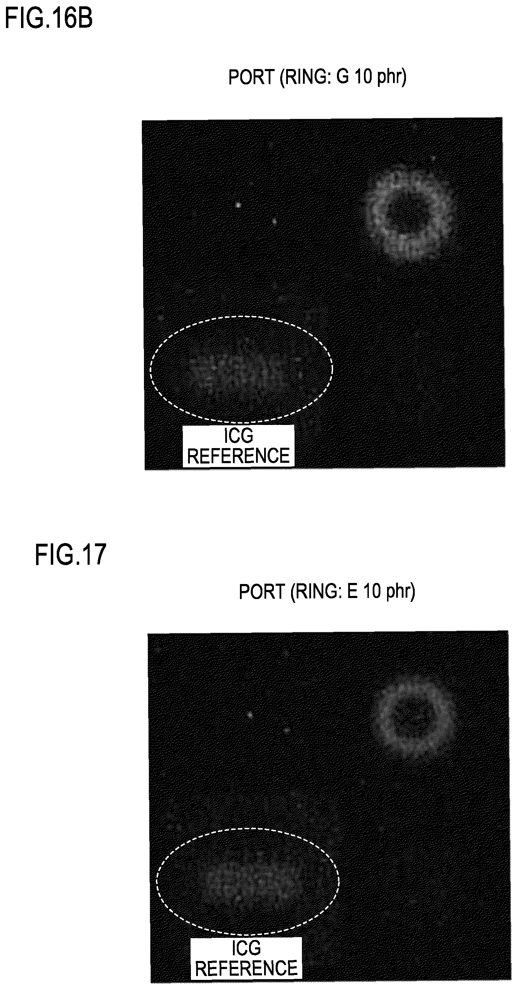

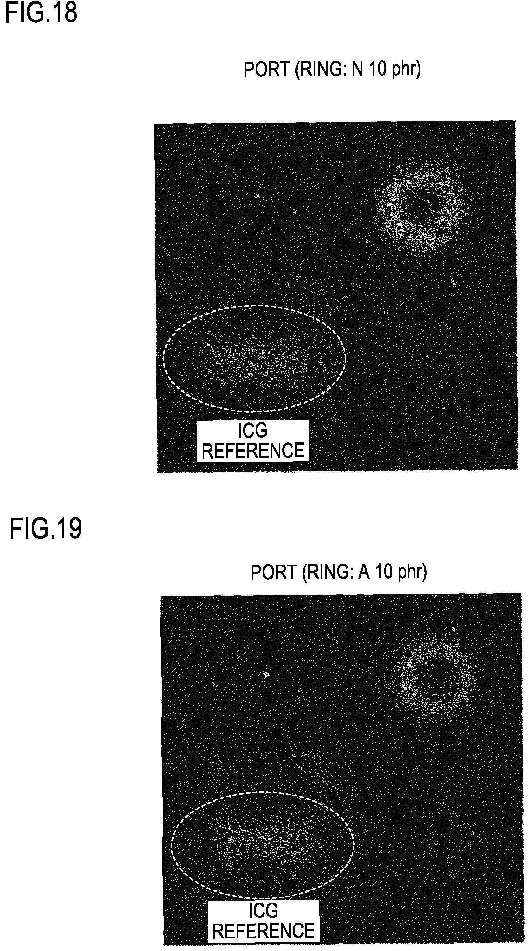

FIG. 16B is a near-infrared image obtained when the CV port 10 and the comparison port 10A of FIG. 16A are irradiated with near-infrared light, and illustrates a near-infrared image in a case where the index member 13 of the CV port 10 is molded by mixing the G solution with epoxy resin at a ratio of 10 phr. FIGS. 17 to 19 respectively illustrate near-infrared images in cases where the index members 13 of the CV ports 10 are molded by mixing the E solution, the N solution, and the A solution, respectively, with epoxy resin at a ratio of 10 phr each.

As illustrated in FIGS. 16B, and 17 to 19, it was found that light emission luminance that is the same as or more than that of the ICG reference card was able to be obtained when the ring-like the index member 13 is molded using any one of the G solution, the E solution, the N solution, and the A solution.

### <Additional Remarks>

The composition for fluorescent labeling, the fluorescent probe, the injection agent, the syringe filling, the medical apparatus, the medical fiber material, the method for producing composition for fluorescent labeling, and the method for producing medical fiber material, which are described above in the present embodiment, can be expressed as the following additional remarks.

A composition for fluorescent labeling according to Additional Remark (1) contains:
a compound (A) expressed by the following formula (1); and
a hydroxyl group-containing organic compound (B). 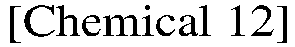 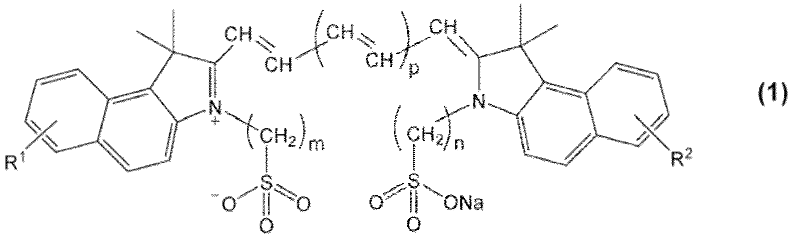 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

A composition for fluorescent labeling according to Additional Remark (2) is the composition for fluorescent labeling according to Additional Remark (1), in which the hydroxyl group-containing organic compound (B) contains an alcoholic hydroxyl group-containing compound (B1).

The composition for fluorescent labeling according to Additional Remark (3) is the composition for fluorescent labeling according to Additional Remark (2), in which the alcoholic hydroxyl group-containing compound (B1) contains an aliphatic alcohol.

The composition for fluorescent labeling according to Additional Remark (4) is the composition for fluorescent labeling according to Additional Remark (2) or (3), which contains:
0.0000001 to 10 wt% of the compound (A); and
1 to 90 wt% of the alcoholic hydroxyl group-containing compound (B1).

The composition for fluorescent labeling according to Additional Remark (5) is the composition for fluorescent labeling according to Additional Remark (2) or (3), which contains 97 to 99.99998 mass parts of the alcoholic hydroxyl group-containing compound (B1), with respect to the total 100 mass parts of the compound (A) and the alcoholic hydroxyl group-containing compound (B1).

The composition for fluorescent labeling according to Additional Remark (6) is the composition for fluorescent labeling according to any one of Additional Remarks (1) to (5), which contains substantially no dimethyl sulfoxide.

The composition for fluorescent labeling according to Additional Remark (7) is the composition for fluorescent labeling according to any one of Additional Remarks (1) to (6), which further contains at least one selected from a group consisting of olefin resin, epoxy resin, vinyl chloride resin, fluorocarbon resin, polycarbonate resin, polyamide resin, ABS resin, acetal resin, acrylic resin, and silicone resin, as the resin (C).

The composition for fluorescent labeling according to Additional Remark (8) is the composition for fluorescent labeling according to any one of Additional Remarks (1) to (7), which contains polyurethane resin.

The composition for fluorescent labeling according to Additional Remark (9) is the composition for fluorescent labeling according to any one of Additional Remarks (1) to (8), which is a solid or semisolid state at 25 degrees Celsius and a relative humidity of 50%.

A fluorescent probe according to Additional Remark (10) contains the composition for fluorescent labeling according to any one of Additional Remarks (1) to (9).

An injection agent according to Additional Remark (11) is an injection agent for labeling an object existing on mucous membrane or skin, which contains the composition for fluorescent labeling according to any one of Additional Remarks (1) to (9).

The injection agent according to Additional Remark (12) is the injection agent according to Additional Remark (11), which further contains at least one of sodium alginate and sodium hyaluronate, as a (D) component.

The injection agent according to Additional Remark (13) is the injection agent according to Additional Remark (12), which contains the (D) component at total 0.01 to 10 wt%.

A syringe filling according to Additional Remark (14) includes:
the composition for fluorescent labeling according to any one of Additional Remarks (1) to (9); and
a syringe filled with the composition for fluorescent labeling.

A medical apparatus according to Additional Remark (15) contains the composition for fluorescent labeling according to any one of Additional Remarks (1) to (9).

The medical apparatus according to Additional Remark (16) is the medical apparatus according to Additional Remark (15), which is one selected from a group consisting of a stent, a tube, a catheter, a clip, a 3D printer filament, and a resin fiber material.

A medical fiber material according to Additional Remark (17) contains:
a compound (A) expressed by the following formula (1); and
a fiber material containing a hydroxyl group-containing organic compound (B). 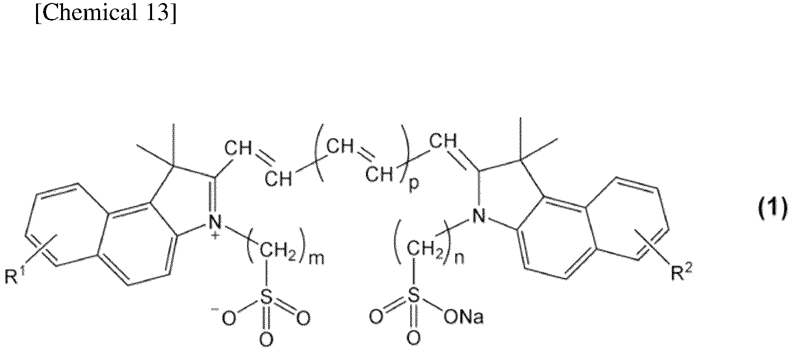
where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

The medical fiber material according to Additional Remark (18) is the medical fiber material according to Additional Remark (17), in which the hydroxyl group-containing organic compound (B) contains cellulose (B2).

A method for producing composition for fluorescent labeling according to Additional Remark (19) comprises a mixing step of mixing a compound (A) expressed by the following formula (1) and a hydroxyl group-containing organic compound (B). 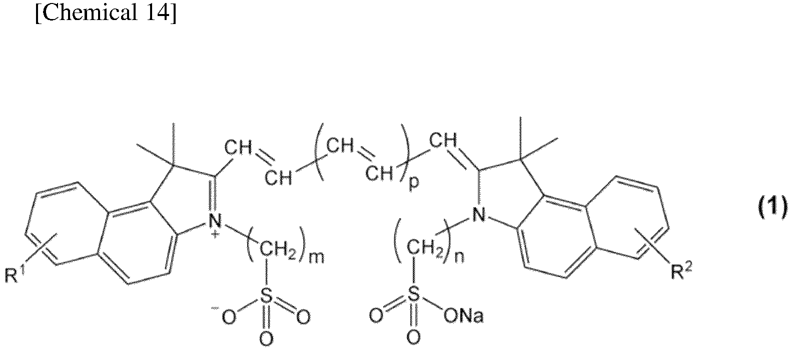 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

The method for producing composition for fluorescent labeling according to Additional Remark (20) is the method for producing composition for fluorescent labeling according to Additional Remark (19), in which the mixing step includes mixing 0.0000001 to 10 wt% of the compound (A) with 1 to 90 wt% of an alcoholic hydroxyl group compound (B1) as the hydroxyl group-containing organic compound (B).

A method for producing medical fiber material according to Additional Remark (21) includes:
an impregnation step of allowing a fiber material containing a hydroxyl group-containing organic compound (B) to be impregnated or sprayed with solution containing a compound (A) expressed by the following formula (1); and
a mordanting step of using a mordant for fixing the compound (A) to the fiber material. 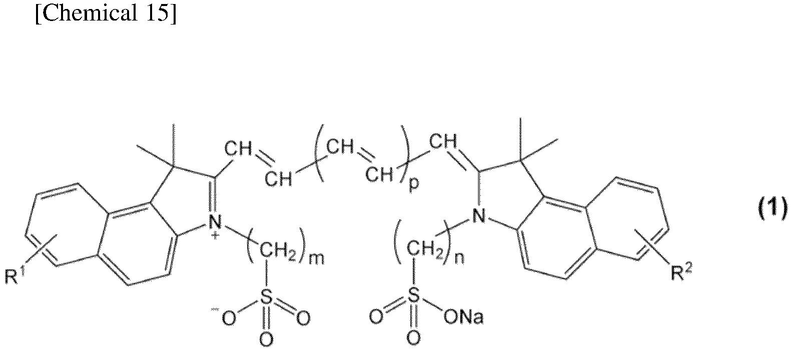 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

The method for producing medical fiber material according to Additional Remark (22) is the method for producing medical fiber material according to Additional Remark (21), in which the hydroxyl group-containing organic compound (B) contains cellulose (B2).

The method for producing medical fiber material according to Additional Remark (23) is the method for producing medical fiber material according to Additional Remark (21) or (22), which further includes a protein processing step for allowing the fiber material to be impregnated with a protein product.

The medical apparatus according to Additional Remark (24) is the medical apparatus according to Additional Remark (15), in which
the medical apparatus is a central venous port,
the central venous port includes a septum, a housing part configured to hold the septum, and an index member disposed inside the housing part and outside the septum, and
the index member contains the composition for fluorescent labeling.

The medical apparatus according to Additional Remark (25) is the medical apparatus according to Additional Remark (24), in which the index member has a shape surrounding the septum.

Although the embodiment of the present invention is described above, the scope of the present invention is not limited to this, and the present invention can be implemented by expanding or modifying within the scope without deviating from the spirit of the invention.

### Industrial Applicability

The present invention can be used for biological fluorescence imaging, for example.

### List of Reference Signs

10 CV port (central venous port)
11 septum
12 housing part
13 index member

## Claims

1. A composition for fluorescent labeling, containing:
a compound (A) expressed by the following formula (1); and
a hydroxyl group-containing organic compound (B). 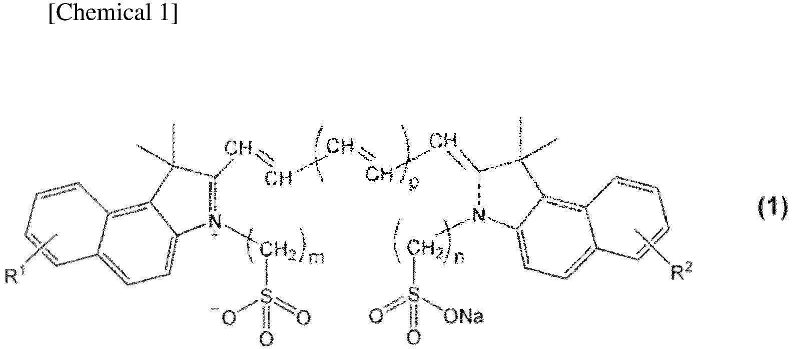 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

2. The composition for fluorescent labeling according to claim 1, wherein the hydroxyl group-containing organic compound (B) contains an alcoholic hydroxyl group-containing compound (B1).

3. The composition for fluorescent labeling according to claim 2, wherein the alcoholic hydroxyl group-containing compound (B1) contains an aliphatic alcohol.

4. The composition for fluorescent labeling according to claim 2, containing:
0.0000001 to 10 wt% of the compound (A); and
1 to 90 wt% of the alcoholic hydroxyl group-containing compound (B1).

5. The composition for fluorescent labeling according to claim 2, containing 97 to 99.99998 mass parts of the alcoholic hydroxyl group-containing compound (B1), with respect to the total 100 mass parts of the compound (A) and the alcoholic hydroxyl group-containing compound (B1).

6. The composition for fluorescent labeling according to claim 1, containing substantially no dimethyl sulfoxide.

7. The composition for fluorescent labeling according to claim 1, further containing at least one selected from a group consisting of olefin resin, epoxy resin, vinyl chloride resin, fluorocarbon resin, polycarbonate resin, polyamide resin, ABS resin, acetal resin, acrylic resin, and silicone resin, as the resin (C).

8. The composition for fluorescent labeling according to claim 1, containing polyurethane resin.

9. The composition for fluorescent labeling according to claim 1, being a solid or semisolid state at 25 degrees Celsius and a relative humidity of 50%.

10. A fluorescent probe containing the composition for fluorescent labeling according to claim 1.

11. An injection agent for labeling an object existing on mucous membrane or skin, containing the composition for fluorescent labeling according to claim 1.

12. The injection agent according to claim 11 further containing at least one of sodium alginate and sodium hyaluronate, as a (D) component.

13. The injection agent according to claim 12, containing the (D) component at total 0.01 to 10 wt%.

14. A syringe filling comprising:
the composition for fluorescent labeling according to claim 1; and
a syringe filled with the composition for fluorescent labeling.

15. A medical apparatus containing the composition for fluorescent labeling according to claim 1.

16. The medical apparatus according to claim 15, being one selected from a group consisting of a stent, a tube, a catheter, a clip, a 3D printer filament, and a resin fiber material.

17. A medical fiber material containing:
a compound (A) expressed by the following formula (1); and
a fiber material containing a hydroxyl group-containing organic compound (B). 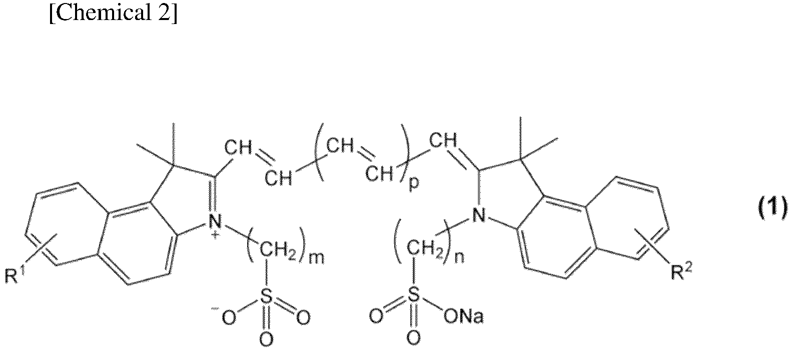 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

18. The medical fiber material according to claim 17, wherein the hydroxyl group-containing organic compound (B) contains cellulose (B2).

19. A method for producing composition for fluorescent labeling, the method comprising a mixing step of mixing a compound (A) expressed by the following formula (1) and a hydroxyl group-containing organic compound (B). 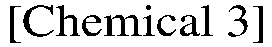 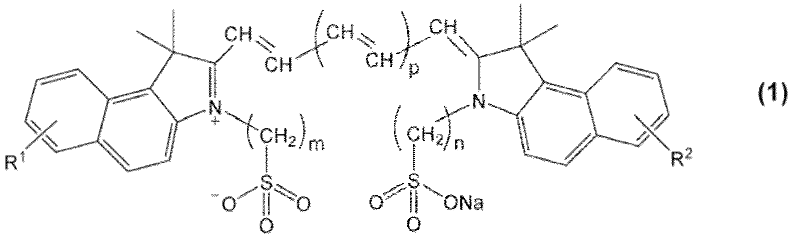 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

20. The method for producing composition for fluorescent labeling according to claim 19, wherein the mixing step includes mixing 0.0000001 to 10 wt% of the compound (A) with 1 to 90 wt% of an alcoholic hydroxyl group compound (B1) as the hydroxyl group-containing organic compound (B).

21. A method for producing medical fiber material, the method comprising:
an impregnation step of allowing a fiber material containing a hydroxyl group-containing organic compound (B) to be impregnated or sprayed with solution containing a compound (A) expressed by the following formula (1); and
a mordanting step of using a mordant for fixing the compound (A) to the fiber material. 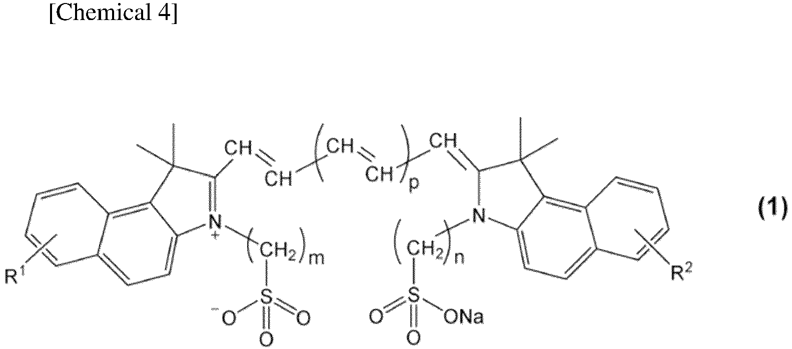 where, R¹ and R² independently represent hydrogen, an alkyl group of carbon number 1 to 13, an alkoxy group of carbon number 1 to 13, or a sulfonate group, p represents an integer of 1 to 5, and m and n independently represent an integer of 1 to 12.

22. The method for producing medical fiber material according to claim 21, wherein the hydroxyl group-containing organic compound (B) contains cellulose (B2).

23. The method for producing medical fiber material according to claim 21, further comprising a protein processing step for allowing the fiber material to be impregnated with a protein product.

24. The medical apparatus according to claim 15, wherein
the medical apparatus is a central venous port,
the central venous port includes a septum, a housing part configured to hold the septum, and an index member disposed inside the housing part and outside the septum, and
the index member contains the composition for fluorescent labeling.

25. The medical apparatus according to claim 24, wherein the index member has a shape surrounding the septum.
